# EUROPÄISCHE PATENTSCHRIFT

(11) **EP 0 560 135 B1**
(45) Veröffentlichungstag und Bekanntmachung des Hinweises auf die Patenterteilung: **17.05.1995**
(21) Anmeldenummer: 93102938.3
(22) Anmeldetag: 25.02.1993
(51) Int. Cl.: A61B 17/06

(54) **Chirurgisches Nahtmaterial**
Surgical suture
Suture chirurgicale

(30) Priorität: 12.03.1992 DE 9203333 U; 05.02.1993 DE 4303374
(43) Veröffentlichungstag der Anmeldung: 15.09.1993
(73) Patentinhaber: Richard Wolf GmbH, 75438 Knittlingen (DE)
(72) Erfinder: Boebel, Manfred, W-7136 Oetisheim (DE); Klemm, Bernd, W-7801 Umkirch (DE)
(74) Vertreter: Patent- und Rechtsanwaltssozietät, Schmitt, Maucher & Börjes

(56) Entgegenhaltungen:
- WO-A-90/14795
- GB-A- 2 157 180
- US-A- 3 985 138
- US-A- 5 002 563
- US-A- 5 127 413

## Beschreibung

Die Erfindung betrifft ein chirurgisches Nahtmaterial mit einem Faden und gegebenenfalls einer Nadel an zumindest einem Fadenende gemäß den Oberbegriffen der unabhängigen Ansprüche 1, 4, 15 und 20 wie es beispielsweise aus der US-A-5 002 563 bekannt ist.

Chirurgisches Nahtmaterial wird als abgelängter, vorkonfektionierter Faden bereitgestellt, der an seinem einen Ende oder auch an beiden Enden meist mit einer chirurgischen Nadel fest verbunden ist. Derartiges Nahtmaterial wird unter anderem als separat verpackter Einzelfaden angeboten, wobei der Faden in der Verpackung gewöhnlich platzsparend aufgewickelt ist.

Insbesondere ein aus monofilem Kunststoffmaterial bestehender Faden eines chirurgischen Nahtmaterials ist vergleichsweise hart und elastisch, so daß er auch nach einer Längsstreckung unkontrolliert in eine undefinierte Rückformung zurückfällt, durch die die Handhabung des vorbekannten Nahtmaterials insbesondere unter beengten Platzverhältnissen erschwert werden kann.

Derartiges Nahmaterial wird auch bei endoskopischen intrakorporalen Operationen verwendet, bei denen sowohl das Arbeitsende der benötigten chirurgischen Instrumente wie auch das chirurgische Nahtmaterial über einzelne Trokarhülsen in das Körperinnere des Patienten eingeführt werden. Um auch bei solchen Operationen das Gewebe mit einer chirurgischen Naht versorgen und dabei einen instrumentellen intrakorporalen chirurgischen Knoten anbringen zu können, wird das zu versorgende Gewebe mit der Nadel durchstochen und der daran anhängende Faden mit einem Faden- oder Nadelhalter erfaßt. Der Faden kann anschließend um einen zweiten Faden- oder Nadelhalter gelegt werden, der dabei seinerseits eine kreisförmige Bewegung vollzieht und mit seinem Schaft den Faden so führt, daß dieser seine Zangenmaulteile spiralförmig umschlingt. Mit diesen Zangenmaulteilen wird sodann das gegenüberliegende Fadenende erfaßt und durch die spiralförmige Umschlingung gezogen. Zieht man nun mit jeweils einem Faden- oder Nadelhalter an beiden Fadenenden, so ist der sogenannte Grundknoten gelegt. Um diesen Grundknoten in seiner Lage zu halten oder die einmal erzeugte Spannung zu sichern, wird auf die gleiche Weise über dem Grundknoten ein sogenannter Endknoten fest geschlungen.

Während bei eröffnetem Abdomen sowie ausreichend langen Fäden des chirurgischen Nahtmaterials ein chirurgischer Knoten auf die oben beschriebene Weise noch vergleichsweise einfach hergestellt werden kann, ist diese Technik bei geschlossenem Abdomen und über Trokarhülsen eingeführten Instrumenten nur sehr schwer durchführbar, weil die Fäden entweder durch die bereits erwähnte Härte und Elastizität und/oder bedingt durch die oft vertikale Stellung der Instrumente bei intraabdominalen endoskopischen Operationen häufig vom Faden- oder Nadelhalter abrutschen und sich in ihre Ausgangslage zurückbewegen. Die über Trokarhülsen in das Bauchinnere eingeführten Instrumente können nur begrenzt entgegen der Abrutschbewegung des Fadens nachgeführt werden, zumal der Raum für Nachfolgebewegungen bei endoskopischen Operationen sehr beschränkt ist.

Vor allem bei besonders steifem monofilem Fadenmaterial, wie es bei endoskopischen Operationen häufig verwendet wird, kann bereits der Faden des Nahtmaterials zum Knoten nur schwer um die Zangenmaulteile geschlungen werden. Gelingt dies doch, so muß der Faden oft mit den Instrumenten unter Zug gehalten werden, um sein Abrutschen vom Faden- oder Nadelhalter zu verhindern; diese Zugbelastung des Fadens aber kann zum Einreißen des zu versorgenden Gewebes führen.

Es besteht daher insbesondere die Aufgabe, ein chirurgisches Nahtmaterial zu schaffen, dessen Faden auch auf engstem Raum schnell und sicher zu einem chirurgischen Knoten geformt werden kann. Dabei soll das erfindungsgemäße Nahtmaterial insbesondere die Bildung eines sicheren Knotens begünstigen und mit möglichst geringem Aufwand gestatten.

Diese aufgabe wird gelöst durch die in den Ansprüche 1, 4, 15 und 20 angegebenen Kennzeichnenden Merkmale.

Die erfindungsgemäße Lösung dieser Aufgabe besteht insbesondere darin, daß das Nahtmaterial in zumindest einem ersten vorgeformten Teilbereich seiner Längserstreckung wenigstens eine eigenstabile Windung, Schlinge oder dergleichen Vorformung aufweist, durch die ein weiterer Teilbereich des Fadens oder ein Fadenende zur Schlaufen- und/oder Knotenbildung durchfädelbar oder durchführbar ist. Das erfindungsgemäße Nahtmaterial weist in zumindest einem ersten vorgeformten Teilbereich seiner Längserstreckung wenigstens eine Windung, Schlinge oder dergleichen Vorformung auf, durch die beispielsweise auch die Zangenmaulteile oder Zangenarme eines Faden- oder Nadelhalters durchführbar sind. Nach Durchführen dieser Zangenmaulteile kann das gegenüberliegende Fadenende mit dem Faden- oder Nadelhalter ergriffen und durch die Vorformung geführt oder gezogen werden, so daß der Grundknoten oder der Endknoten eines chirurgischen Knotens entsteht. Statt die Zangenmaulteile durch die Vorformungen des Nahtmaterials durchzuführen, kann beispielsweise auch ein Fadenende dort hindurchgefädelt werden. Mit Hilfe des vorgeformten Teilbereiches des erfindungsgemäßen Nahtmaterials ist eine einfache Schlaufen- oder Knotenbildung möglich, ohne daß ein gerade unter beengten Raumverhältnisses aufwendiges und teils auch nur schwer erreichbares Umschlingen des Fadens um die Zangenmaulteile durch Kreisbewegungen des Faden- oder Nadelhalters erforderlich wäre.

Erfindungsgemäß weist das Nahtmaterial mehrere, vorzugsweise zwei voneinander beabstandete vorgeformte Teilbereiche auf, deren wendel- oder spiralförmigen Vorformungen gegensinnige Drehrichtungen aufweisen. Bei einer solchen Ausführungsform lassen sich der Grundknoten und der Endknoten als Schifferknoten ausführen, bei dem eines der Fadenenden einmal vorwärts und einmal rückwärts um den anderen Fadenteil herumgeschlungen wird.

Ist demgegenüber nur ein sogenannter Weiber- oder Frauenknoten gewünscht, bei dem eines der beiden Fadenteile zweimal um den anderen Teil geknotet wird, und der sich durch ein gewisses Nachgeben des Knotens auszeichnet, so ist lediglich nur ein wendel- oder spiralförmiger Teilbereich nötig, wenn das Nahtmaterial zumindest in seinem vorgeformten Teilbereich aus elastischem Material hergestellt ist. Denn nach dem Knoten des Grundknotens und Festziehen der Fadenenden fällt der vorgeformte Teilbereich dank der Elastizität des Nahtmaterials in diesem Teilbereich in seine wendel- oder spiralförmige Formgebung zurück, so daß auch der Endknoten durch Durchziehen des gegenüberliegenden Fadenendes durch die Vorformung gebildet werden kann.

Vorteilhaft ist es, wenn die wendel- oder spiralförmige Vorformung zumindest eines Teilbereiches, vorzugsweise von zwei Teilbereichen des Nahtmaterials jeweils mehrere, zweckmäßigerweise zwei Windungen oder dergleichen Vorformungen aufweist. Somit kann der Grundknoten und gegebenenfalls auch der Endknoten zu einem doppelt geschlungenen, sogenannten chirurgischen Knoten gelegt werden.

Um bei dem erfindungsgemäßen Nahtmaterial auf einfache Weise einen Knoten bilden und dabei die Zangenarme eines Faden- oder Nadelhalters durch dessen Vorformungen durchführen zu können, ist es zweckmäßig, wenn jeder wendel- oder spiralförmig vorgeformte Teilbereich des Nahtmaterials zum Durchführen eines chirurgischen Fadenhalters in seinem lichten Durchmesser an die Außenabmessungen dieses Fadenhalters im Bereich von dessen Zangenmaulteilen angepaßt ist.

Eine Weiterbildung gemäß der Erfindung von eigener schutzwürdiger Bedeutung sieht vor, daß das Nahtmaterial zumindest in einem ersten vorgeformten Teilbereich seiner Längserstreckung wenigstens zwei ineinander übergehende Windungen, Schlingen oder dergleichen Vorformungen aufweist, welche gegensinnige Drehrichtungen haben, daß durch mindestens eine dieser Windungen oder dergleichen Vorformungen ein weiterer Teilbereich des Fadens oder ein Fadenende zur Schlaufen- und/oder Knotenbildung durchführbar ist und daß die Zentralachsen dieser benachbarten gegensinnigen Windungen oder dergleichen Vorformungen voneinander beabstandet sind. Um auf einfache Weise mit Hilfe des erfindungsgemäßen Nahtmaterials einen Knoten oder eine Schlaufe bilden zu können, kann durch zumindest eine der Windungen oder dergleichen Vorformungen beispielsweise das gegenüberliegende Fadenende durchgezogen werden. Dabei ist es ein besonderer Vorteil dieser erfindungsgemäßen Weiterbildung, daß durch die gegensinnige Drehrichtung seiner Windungen, Schlingen oder dergleichen Vorformungen einer unerwünschten Verdrehung des Fadens entgegengewirkt wird, so daß ein spannungsfreier Knoten entsteht und keine Lockerung des gelegten Knotens bei verdrehten Fäden eintreten kann.

Beim Durchführen eines Fadenendes durch eine vorgeformte Windung und Zuziehen des Knotens kann nämlich eine axiale Verdrehung des Fadens entstehen, die vor allem bei steifem Fadenmaterial und beim Knoten auf engem Raum die korrekte Lage des Knotens im Bereich des Knotensitzes und damit die Haltefunktion des Knotens verschlechtern kann. Auch kann die Verdrehung des Fadens beim chirurgischen Knoten im Bereich des Knotensitzes sogar zu einer "Luftschlaufenbildung" führen, so daß bereits die Haltefunktion des Grundknotens unzureichend ist. Ein eventuelles späteres Nachgeben des Knotens kann zu einer Dehiszenz des adaptierten Gewebes und/oder eventuell auch zu einer Nachblutung bei Blutgefäßen führen. Dieser für die Knotensicherheit ungünstige Effekt wirkt sich umso stärker aus, je kürzer der Faden gestaltet ist.

Da bei dieser Weiterbildung der Erfindung dieser nachteilige Effekt der Verdrehung des Fadens kaum auftreten kann, ist es möglich, auch mit sehr kurzen Fäden noch einen spannungsfreien Knoten zu erzeugen. Da die Windungen oder dergleichen Vorformungen des erfindungsgemäßen Nahtmaterials ineinander übergehen, kann dieses auch in seinem vorgeformten Teilbereich vergleichsweise kurz ausgestaltet werden. Beides kommt einer einfachen Handhabung entgegen, beispielsweise wenn im Rahmen einer intrakorporalen Operation das Fadenmaterial durch die Trokarhülse in das Körperinnere des Patienten eingeführt werden muß. Gleichzeitig wird durch diese vergleichsweise kurze Ausgestaltung des erfindungsgemäßen Nahtmaterials ein unnötiger Aufwand beim Zuziehen eines Knotens vermieden, da sonst bei längeren Fäden das Durchführen des Fadenendes und Zuziehen des Knotens mitunter viel Raum für die Instrumentenbewegung oder sogar mehrmaliges Nachfassen erfordert. Dabei sind die Zentralachsen der benachbarten gegensinnigen Windungen oder dergleichen Vorformungen voneinander beabstandet, so daß zur Bildung eines Schifferknotens ein Fadenende zunächst nur durch die eine Windung des vorgeformten Teilbereiches gezogen und der Grundknoten gelegt wird, um anschließend eventuell mit Hilfe der anderen, eine gegensätzliche Drehrichtung aufweisenden Windung den entgegengesetzt geschlungenen Endknoten zu legen.

Ist demgegenüber ein vergleichsweise lockerer Knotensitz gewünscht, so muß zur Bildung eines Weiber- oder Frauenknotens lediglich ein und dieselbe Windung für Grund- und Endknoten verwendet werden.

Um die Zangenmaulteile eines Fadenhalters oder auch nur um ein Fadenende gut durch die Windungen oder dergleichen Vorformungen des Nahtmaterials führen zu können, ist es zweckmäßig, wenn jeweils zwei einander benachbarte Windungen oder dergleichen Vorformungen mit gegensinnigen Drehrichtungen in etwa einer Ebene angeordnet sind.

Eine bevorzugte Ausführungsforn gemäß der Erfindung sieht vor, daß jeweils zwei einander benachbarte Windungen oder dergleichen Vorformungen mit gegensinnigen Drehrichtungen eine etwa acht-förmige Vorformung oder Doppelschlaufe bilden. Eine derartige acht-förmige Doppelschlaufe benötigt eine vergleichsweise kurze Fadenlänge und vereinfacht die Handhabung des erfindungsgemäßen Nahtmaterials wesentlich.

Um einer Verdrehung des Fadens wegen der Windungen oder dergleichen Vorformungen entgegenzuwirken, ist es vorteilhaft, wenn von zwei einander benachbarten Windungen oder dergleichen Vorformungen mit gegensinnigen Drehrichtungen der Einlaufabschnitt der einen Windung und der Auslaufabschnitt der anderen Windung auf gegenüberliegenden Seiten ihres gemeinsamen Übergangsbereiches angeordnet sind. Dabei ist unter dem Einlaufabschnitt der einen Windung und dem Auslaufabschnitt der anderen Windung der in derselben Fadenrichtung vordere bzw. hintere Windungsabschnitt der jeweiligen Windungen oder dergleichen Vorformungen zu verstehen, während als Übergangsbereich der zwischen den benachbarten Windungen vorgesehene Materialabschnitt bezeichnet ist.

Eine Verdrehung des erfindungsgemäßen Nahtmaterials wird praktisch vollständig vermieden, wenn jeder Windung oder dergleichen Vorformung des vorgeformten Teilbereiches mit der einen Drehrichtung eine weitere Windung oder Vorformung mit entgegengesetzter Drehrichtung zugeordnet ist.

Zweckmäßig ist es, wenn der vorgeformte Teilbereich des Nahtmaterials mehr als zwei ineinander übergehende Windungen oder dergleichen Vorformungen aufweist, wenn jeweils benachbarte Vorformungen gegensinnige Drehrichtungen haben und wenn die Zentralachsen der Vorformungen mit gleicher Drehrichtung etwa koaxial zueinander angeordnet sind. Bei dieser Ausführungsform weist der vorgeformte Teilbereich des erfindungsgemäßen Nahtmaterials zumindest zwei übereinander angeordnete Windungen, Schlingen oder dergleichen mit gleicher Drehrichtung neben einer weiteren Vorformung mit entgegengesetzter Drehrichtung auf. Wird nun ein Fadenende durch die beiden Windungen mit gleicher Drehrichtung gezogen, so kann eine Doppelumschlingung gelegt werden, wie sie beispielsweise für den sogenannten chirurgischen Knoten notwendig ist. Dabei sieht eine besonders einfache Ausführungsform gemäß der Erfindung vor, daß der vorgeformte Teilbereich des Nahtmaterials drei ineinander übergehende Windungen oder dergleichen Vorformungen hat, wobei zwei Vorformungen mit gleicher Drehrichtung eine Vorformung mit dazu gegensinniger Drehrichtung zwischen sich einschließen. Mit Hilfe einer solchen Ausführungsform kann ein chirurgischer Knoten gebildet werden, wobei mittels der zwei, etwa koaxial angeordneten Windungen oder dergleichen mit gleicher Drehrichtung ein doppelt geschlungener Grundknoten und mittels der Windung mit dazu gegensinniger Drehrichtung ein einfacher Endknoten gelegt werden kann.

Um bei der Verwendung des erfindungsgemäßen Nahtmaterials die Position des vorgeformten Teilbereiches in seiner Winkelstellung zum abgehenden Faden oder angrenzenden Fadenabschnitt zu verdeutlichen und um die Handhabung dieses Nahtmaterials noch zusätzlich zu vereinfachen ist es zweckmäßig, wenn zumindest einer, vorzugsweise beide der an den vorgeformten Teilbereich angrenzenden Abschnitte des Nahtmaterials quer, insbesondere rechtwinklig zu der durch die benachbarten Windungen oder dergleichen und/oder die Doppelschlaufen gebildeten Ebene abgewinkelt sind.

Die oben beschriebene Ausführungsform des erfindungsgemäßen Nahtmaterials mit ihren acht-förmigen Doppelschlaufen stellt wegen ihrer einfachen Ausgestaltung und Handhabung und wegen der Möglichkeit, auch mit sehr kurzen Fäden noch einen sicheren, spannungsfreien Knoten legen zu können, die bevorzugte Ausführungsform dar. Nach einem weiterbildenden Vorschlag gemäß der Erfindung, für den eigenständiger Schutz beansprucht wird, ist jedoch auch vorgesehen, daß zwei einander benachbarte Windungen oder dergleichen Vorformungen oder zwei benachbarte vorgeformte Teilbereiche mit gegensinnigen Drehrichtungen brillenförmig zueinander angeordnet sind. Dabei können die beiden benachbarten Teilbereiche jeweils auch wendel- oder spiralförmig vorgeformt sein. Dabei wird zumindest bei einer brillenförmigen Anordnung zweier benachbarter Windungen oder dergleichen Vorformungen mit gegensinnigen Drehrichtungen einer Verdrehung des Nahtmaterials entgegengewirkt, wenn der Einlaufabschnitt der einen Windung und der Auslaufabschnitt der anderen Windung auf derselben Seite ihres gemeinsamen Übergangsbereiches angeordnet sind.

Um nach dem Anlegen des Grundknotens den Endknoten in entgegengesetzte Richtung zu einem Schifferknoten oder in gleicher Richtung zu einem Weiber- oder Frauenknoten umschlingen zu können und um dazu beispielsweise jeweils die Windungen oder dergleichen Vorformungen mit der entsprechenden Drehrichtung erkennen zu können, ist es vorteilhaft, wenn die vorgeformten Teilbereiche des Nahtmaterials, insbesondere die Windungen oder dergleichen Vorformungen mit derselben Drehrichtung und/oder die zwischen zwei benachbarten vorgeformten Teilbereichen vorgesehenen Abschnitte des Nahtmaterials und/oder die zu den Vorformungen hin- und/oder wegführenden Abschnitte des Nahtmaterials eine Signaleinfärbung, Signalbeschichtung oder dergleichen Signalmarkierung aufweisen. Dabei können beispielsweise entweder nur die Windungen oder Vorformungen mit der einen Drehrichtung eine derartige Signalmarkierung aufweisen oder aber sowohl die Windungen mit der einen Drehrichtung als auch die mit der anderen Drehrichtung, wobei im letzteren Fall die Windungen oder Vorformungen je nach Drehrichtung eine bestimmte unterschiedliche Signalmarkierung aufweisen sollten. Eine solche, von den Windungen oder den vorgeformten Teilbereichen abweichende Farbgebung empfiehlt sich auch für den zwischen zwei benachbarten vorgeformten Teilbereichen vorgesehenen Abschnitt des Nahtmaterials beziehungsweise für die zu den Vorformungen hin- und/oder wegführenden Abschnitte des Nahtmaterials.

Die leichte Handhabung und das einfache Anlegen eines Knotens kann bei dem erfindungsgemäßen Nahtmaterial noch dadurch begünstigt werden, daß die Längsachse(n) des vorgeformten Teilbereiches (der vorgeformten Teilbereiche) in Richtung der Längserstreckung des Nahtmaterials angeordnet ist(sind). Somit bildet jeder vorgeformte Teilbereich des Nahtmaterials praktisch eine quer zu seiner Längserstreckung angeordnete Fadenöffnung, durch die auf einfache Weise die beiden Zangenmaulteile eines üblichen Faden- oder Nadelhalters durchgeführt oder das eine der beiden Enden des Nahtmaterials hindurchgefädelt werden können.

Der vorgeformte Teilbereich kann bei dem erfindungsgemäßen Nahtmaterial durch eine entsprechende Beschichtung, Klebung des Naht- oder Fadenmaterials oder Tränkung in einer formstabilisierenden Lösung oder durch dergleichen Behandlung erzeugt und in die gewünschte Formgebung gebracht werden, so daß beispielsweise die aneinander anliegenden benachbarten Bereiche im vorgeformten Teilbereich des erfindungsgemäßen Nahtmaterials miteinander verkleben. Um Grund- und Endknoten hier auf einfache Weise legen zu können, sind bei einer solchen Ausführungsform beispielsweise lediglich zwei vorgeformte Teilbereiche vorzusehen.

Nach einem weiterbildenden Vorschlag gemäß der Erfindung ist jedoch vorgesehen, daß zumindest der vorgeformte Teilbereich des Nahtmaterials aus elastischem Material besteht. Da bei dieser bevorzugten Ausführungsform des erfindungsgemäßen Nahtmaterials zumindest sein vorgeformter Teilbereich aus elastischem Material besteht, das in entspanntem Zustand in seine ursprünglich vorgeformte Form zurückfällt, bilden sich die Windungen oder dergleichen Vorformungen dieses vorgeformten Teilbereiches nach dem Legen des Grundknotens und Entlasten des Fadens erneut und können somit auch für den Endknoten verwendet werden. Außerdem kann das Nahtmaterial zunächst auch durch eine Trokarhülse oder das zu versorgende Gewebe gezogen werden, um anschließend dennoch zumindest bereichsweise die zum Knoten gewünschte Vorformung, beispielsweise die wendel- oder spiralförmige Formgebung, anzunehmen. Das erfindungsgemäße Nahtmaterial ist daher nicht nur bei Eingriffen am eröffneten Abdomen sowie bei Hautnähten einsetzbar, sondern insbesondere auch bei endoskopischen intrakorporalen Operationen.

Um auch die Vorteile unterschiedlicher Naht- oder Fadenmaterialien miteinander kombinieren zu können und um beispielsweise auch einen Knoten mit Hilfe eines unelastischen Fadenmaterials legen zu können, ist nach einem weiterbildenden Vorschlag gemäß der Erfindung von eigener schutzwürdiger Bedeutung vorgesehen, daß der vorgeformte Teilbereich (die vorgeformten Teilbereiche) des Nahtmaterials als Teilstück(e) ausgebildet ist(sind), welche(s) mit dem(den) angrenzenden Fadenabschnitt(en) vorzugsweise mittels eines Steck-, Klemm- oder dergleichen Verbindungselementes insbesondere lösbar verbunden ist. Bei dieser weiterbildenden Ausführungsform gemäß der Erfindung kann das chirurgische Nahtmaterial zweiteilig oder dreiteilig ausgebildet werden und weist beispielsweise den zur Knoten- und/oder Schlaufenbildung vorgeformten Teilbereich aus vorzugsweise elastischem Material auf, der wenigstens an seinem einen freien Ende mit einem Fadenabschnitt aus elastischem oder eventuell auch unelastischem Material insbesondere lösbar verbindbar ist. Dabei kann die Verbindung dieses vorgeformten Teilbereiches an einem seiner beiden Enden mit dem angrenzenden Fadenabschnitt oder mit einem weiteren vorgeformten Teilbereich mittels eines Steck-, Klemm- oder dergleichen Verbindungselementes erfolgen.

In besonderen Anwendungsfällen kann es zweckmäßig sein, wenn der Faden und/oder der vorgeformte Teilbereich (die vorgeformten Teilbereiche) des Nahtmaterials aus einem memory-Metall, insbesondere aus einer Nickel-Titan-Metallegierung, besteht. Dabei kann entweder das gesamte Nahtmaterial im wesentlichen aus einem derartigen memory-Metallfaden bestehen oder aber das erfindungsgemäße Nahtmaterial weist lediglich einen mit dem üblichen Faden beispielsweise lösbar verbundenen, vorgeformten Teilbereich aus einer derartigen memory-Metallegierung auf. Bei derartigen Ausführungsformen kann das erfindungsgemäße Nahtmaterial beispielsweise in einer langgestreckten Form durch eine Trokarhülse oder dergleichen in das Körperinnere eingeführt werden, um dort den Teilbereich aus memory-Metall durch eine Wärme- oder Hitzeeinwirkung in seine eigenstabil vorgeformte Formgebung zu bringen.

Weitere Merkmale der Erfindung ergeben sich aus der folgenden Beschreibung erfindungsgemäßer Ausführungsbeispiele in Verbindung mit den Ansprüchen sowie der Zeichnung. Die einzelnen Merkmale können je für sich oder zu mehreren bei einer Ausführungsform der Erfindung verwirklicht sein.

Es zeigt:
- Fig. 1: ein chirurgisches Nahtmaterial in einer Seitenansicht mit einem Faden sowie einer damit verbundenen Nadel, wobei der Faden in zwei voneinander beabstandeten vorgeformten Teilbereichen jeweils mehrere eigenstabile Windungen aufweist, die eine wendelförmige Vorformung bilden,
- Fig. 2: ein chirurgisches Nahtmaterial, ähnlich dem aus Fig.1,
- Fig. 3: ein chirurgisches Nahtmaterial, dessen Faden zwei spiralförmig vorgeformte und voneinander ebenfalls beabstandete Teilbereiche aufweist,
- Fig. 4: ein chirurgisches Nahtmaterial, dessen Faden in einem vorgeformten Teilbereich seiner Längserstreckung mehrere ineinander übergehende Windungen, Schlingen oder dergleichen Vorformungen aufweist, welche gegensinnige Drehrichtungen haben, wobei jeweils zwei benachbarte Vorformungen mit gegensinnigen Drehrichtungen eine etwa acht-förmige Doppelschlaufe bilden,
- Fig. 5 bis 10: das Setzen einer Einzelnaht mit einem Nahtmaterial, ähnlich dem aus Fig.1, welches in zwei voneinander beabstandeten Teilbereichen seiner Längserstreckung wendelförmig vorgeformt ist,
- Fig.11: ein chirurgisches Nahtmaterial, ähnlich dem aus Fig.4, wobei dessen Faden in einem Teilbereich seiner Längserstreckung aus elastischem Material vier vorgeformte und ineinander übergehende Windungen oder dergleichen Vorformungen aufweist, welche zu ihren benachbarten Windungen jeweils eine gegensätzliche Drehrichtung aufweisen und zusammen zwei etwa achtförmige Doppelschlaufen bilden,
- Fig.12: den Faden eines chirurgischen Nahtmaterials mit drei etwa acht-förmigen Doppelschlaufen,
- Fig.13: den Faden eines chirurgischen Nahtmaterials, ähnlich dem aus den Figuren 4,11 und 12, wobei die beide an den vorgeformten Teilbereich angrenzenden Fadenabschnitte im Bereich der äußeren Enden der acht-förmigen Doppelschlaufen etwa rechtwinklig zu der durch die benachbarten Windungen gebildeten Ebene abgewinkelt sind,
- Fig.14: ein chirurgisches Nahtmaterial, dessen außerhalb der Vorformungen angeordneten Fadenabschnitte in Richtung zu dem angrenzenden vorgeformten Teilbereich abgewinkelt sind,
- Fig.15,16: zwei weitere Ausführungsformen eines chirurgischen Nahtmaterials, bei denen die Windungen, Schlingen oder dergleichen Vorformungen der beiden voneinander beabstandeten vorgeformten Teilbereiche im wesentlichen dreieckförmig ausgebildet sind,
- Fig. 17: einen aus zwei Doppelschlaufen gebildeten Teilbereich, wobei diese Doppelschlaufen versetzt zueinander angeordnet sind und
- Fig. 18: den vorgeformten Teilbereich eines Nahtmaterials, wobei der Teilbereich im wesentlichen eine Doppelschlaufe sowie eine sich daran anschließende, aus drei Schlingen gebildete Vorformung hat.

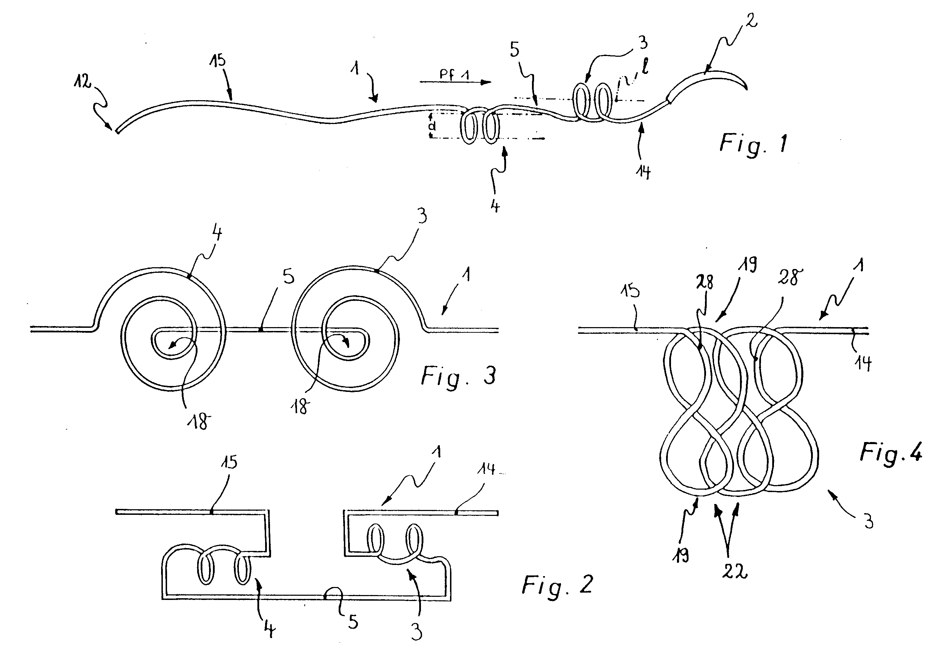

In Fig.1 ist ein chirurgisches Nahtmaterial mit einem Faden 1 dargestellt, der an seinem einen Fadenende mit einer angeformten Nadel 2 verbunden ist. Um das Anlegen von Knoten auch unter beengten Raumverhältnissen wesentlich zu erleichtern, weist der Faden 1 des Nahtmaterials in seiner Längserstreckung zwei vorgeformte Teilbereiche 3,4 auf, die zum Durchführen der beiden Zangenmaulteile eines hier nicht dargestellten Faden- oder Nadelhalters oder zum Durchführen eines Fadenendes jeweils eine wendel- oder schraubenlinienförmige Formgebung haben. Zum Durchführen eines Faden- oder Nadelhalters sind diese Teilbereiche 3,4 in ihrem lichten Durchmesser d an die Außenabmessungen dieses Halters im Bereich von dessen Zangenmaulteilen angepaßt.

Die Teilbereiche 3,4 können grundsätzlich an einer beliebigen Stelle der Längserstreckung des Nahtmaterials angeordnet sein, bevorzugt ist jedoch eine Anordnung dieser Teilbereiche 3,4 im Bereich des Fadens 1, insbesondere - wie hier - im Bereich eines der beiden Fadenenden.

Der gesamte Faden 1 und somit auch dessen Teilbereiche 3,4 sind aus elastischem Material hergestellt, so daß auch nach Durchziehen des Fadens 1 durch eine Trokarhülse oder das zu versorgende Gewebe und einem Strecken des Fadens dieser in seine ursprüngliche, hier gezeigte Form zurückfällt.

Wie aus Fig.1 gut erkennbar ist, sind die auf gegenüberliegenden Seiten des Nahtmaterials angeordneten vorgeformten Teilbereiche 3,4 voneinander beabstandet und weisen gegensinnige Drehrichtungen auf. Nach Durchführen der beiden Zangenarme oder -maulteile eines Faden- oder Nadelhalters durch die wendelförmige Vorformung des Teilbereiches 4 kann das gegenüberliegende Fadenende 12 ergriffen und so durch diesen Teilbereich 4 gezogen werden, daß der Grundknoten entsteht. Ist der Grundknoten gelegt, so fallen die Teilbereiche 3,4 in ihre ursprüngliche, hier gezeigte Form zurück. Um auch den Endknoten über den Grundknoten zu legen, werden die beiden Zangenarme des Faden- oder Nadelhalters nun durch den Teilbereich 3 - ebenfalls in Pfeilrichtung Pf1 - geführt, um wiederum das Fadenende 12 zu ergreifen und durch die wendelförmigen Vorformungen dieses Teilbereiches 3 zu ziehen. Wegen der gegensinnigen Drehrichtung dieser Teilbereiche 3,4 sind hier Grund- und Endknoten als Schifferknoten angelegt, der bei steigendem Fadenzug die beiden gleichartigen, aber entgegengesetzt liegenden Schlaufen der einzelnen Knoten immer mehr zuzieht.

Sollen die Knoten sich als doppelt geschlungener chirurgischer Knoten bilden, so ist es zweckmäßig, wenn die Teilbereiche 3,4 jeweils wendelförmige Vorformungen mit - wie hier - zweckmäßigerweise zwei Windungen aufweisen. Um auch bei einem geradlinig gestreckten Faden die Teilbereiche 3,4 mit den wendel- oder schraubenlinienförmigen Vorformungen erkennen zu können, ist es vorteilhaft, wenn diese und gegebenenfalls auch der zwischen ihnen angeordnete Fadenbereich 5 und/oder der jeweils zu einem vorgeformten Teilbereich hin- oder wegführende Abschnitt des Nahtmaterials eine Signaleinfärbung, Signalbeschichtung oder dergleichen Signalmarkierung aufweisen. Dabei ist es besonders zweckmäßig, wenn die Signalmarkierungen der Teilbereiche 3,4 sowie der übrigen markierten Fadenbereiche sich jeweils voneinander unterscheiden.

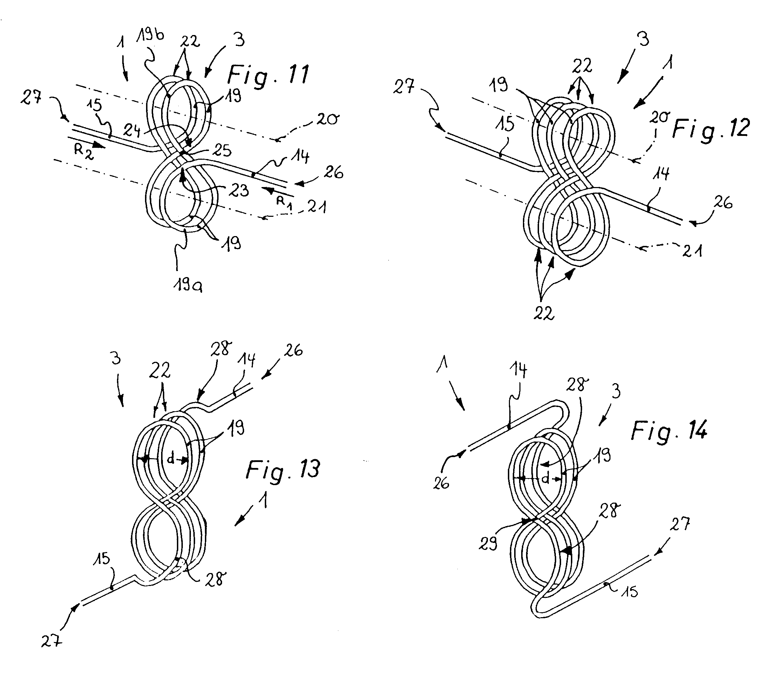

So kann beispielsweise bei der in Figur 1 dargestellten Ausführungsform des Nahtmaterials vorgesehen sein, daß der vorgeformte Teilbereich 3 sowie der zu diesem Teilbereich 3 führende, an die Nadel 2 angrenzende Abschnitt des Fadens eine bestimmte Farbmarkierung aufweist, die die Einfädel- oder Einziehrichtung dieses Teilbereiches 3 markiert, während der Fadenbereich 5 sowie der Teilbereich 4 keine oder eine andere Signalmarkierung trägt. Bei den in Figur 11 und 12 dargestellten Ausführungsformen ist es dagegen ausreichend, nur die in einer Drehrichtung orientierten Windungen 19 sowie den an diese Windungen angrenzenden Fadenabschnitt farblich zu markieren, um dem Anwender die Einfädel- oder Einziehrichtung der in beide Drehrichtungen orientierten Schlaufenteile der Doppelschlaufen 22 anzuzeigen.

Das chirurgische Nahtmaterial kann aus verschiedenen Materialien, - über seine Längserstreckung auch aus unterschiedlichen Werkstoffen, hergestellt werden. Beispielsweise kann der Faden aus einem unelastischen Material bestehen, wobei die vorgeformten Teilbereiche des Fadens durch eine entsprechende Verklebung oder dergleichen Beschichtung der benachbarten Windungen gebildet werden. Möglich ist auch, den Faden 1 des Nahtmaterials aus einer memory-Metallegierung herzustellen, die temperaturabhängig von einer langgestreckten Formgebung in ihre bereichsweise wendelförmigen Vorformungen fällt. Zweckmäßigerweise besteht der Faden 1 des chirurgischen Nahtmaterials zumindest im Bereich der wendel- oder spiralförmig vorgeformten Teilbereiche aus einem elastischen Material. Die wendel- oder spiralförmigen Vorformungen des Fadens in diesen Teilbereichen erlauben ein problemloses Durchfassen der beiden Zangenmaulteile eines üblichen Faden- oder Nadelhalters, so daß das gegenüberliegende Fadenende mit diesen Zangenmaulteilen ergriffen und durch den vorgeformten Teilbereich durchgezogen werden kann.

Ein umständliches Umschlingen des Fadens um den Faden- oder Nadelhalter kann entfallen, ebenso muß eine - wie bisher - umständlich um die Zangenmaulteile des Faden- oder Nadelhalters gelegte Umschlingung nicht mehr unter Zug gehalten werden, um deren Abrutschen vom Faden- oder Nadelhalter zu vermeiden. Auch dadurch kann das Verletzungsrisiko bei dem erfindungsgemäßen Nahtmaterial vermindert werden. Die zum Legen des Knotens benötigte Schlinge kann raumsparend in unmittelbarer Nähe zum späteren Knotensitz angeordnet werden, wobei auch mittels einfacher herkömmlicher Fadenhalter oder Haltezangen und ohne Kreisbewegungen dieser Instrumente zum Erzeugen der Schlinge bei dem erfindungsgemäßen Nahtmaterial der gewünschte Knoten zu legen ist.

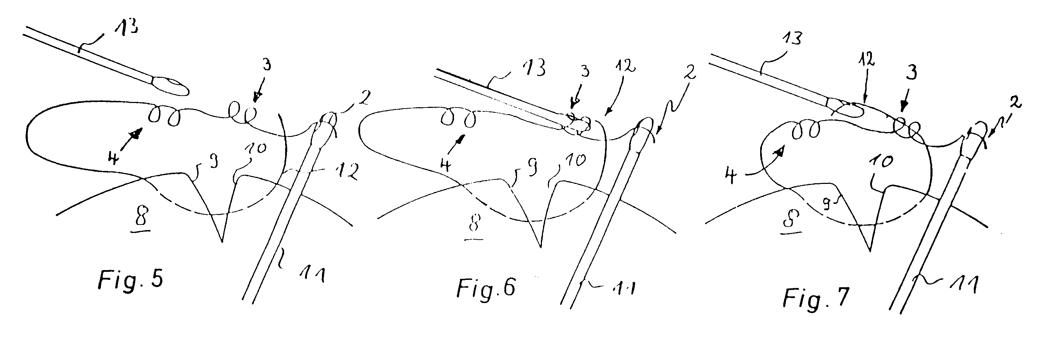

Anhand der Figuren 5 bis 10 wird nachfolgend beschrieben, wie mit dem in Fig.1 dargestellten Nahtmaterial eine Naht mit abschließendem Knoten gebildet wird. In den Figuren 5 bis 10 ist das durch die Naht miteinander zu verbindende Gewebe mit 8 gekennzeichnet, wobei die Ränder 9 und 10 eines hier stark schematisiert dargestellten, im Querschnitt V-förmigen Einschnittes durch die Naht miteinander verbunden werden sollen. In der Stellung nach Fig.5 ist die Nadel 2 mit dem daran befestigten Faden 1 unter Zuhilfenahme eines ersten Nadelhalters 11 durch das Gewebe 8, und zwar zunächst durch den Rand 10 und dann den Rand 9 durchgeführt worden, wobei der Faden 1 so weit durch die zu vernähende Stelle gezogen worden ist, bis nur noch ein für die Knotenbildung ausreichend langes Fadenende 12 des sich auf der der Nadel 2 abgewandten Seite des Nahtmaterials an den Teilbereich 4 anschließenden ungeformten Fadenabschnitt 15 aus dem Gewebe 8 hervorsteht.

Sodann wird, wie in Fig.6 dargestellt ist, mit einem zweiten Nadelhalter 13 durch die zwei wendelförmigen Vorformungen des ersten vorgeformten Teilbereiches 3 von dem der Nadel 2 abgewandten Fadenabschnitt 5 aus durchgriffen und das freie Fadenende 12 mit diesem zweiten Fadenhalter 13 aufgenommen. Danach wird mit dem Nadelhalter 13 das Fadenende 12 durch die wendelförmigen Vorformungen des Teilbereiches 3 - wie in Figur 7 dargestellt - zurückgezogen, wodurch der Grundknoten gebildet wird.

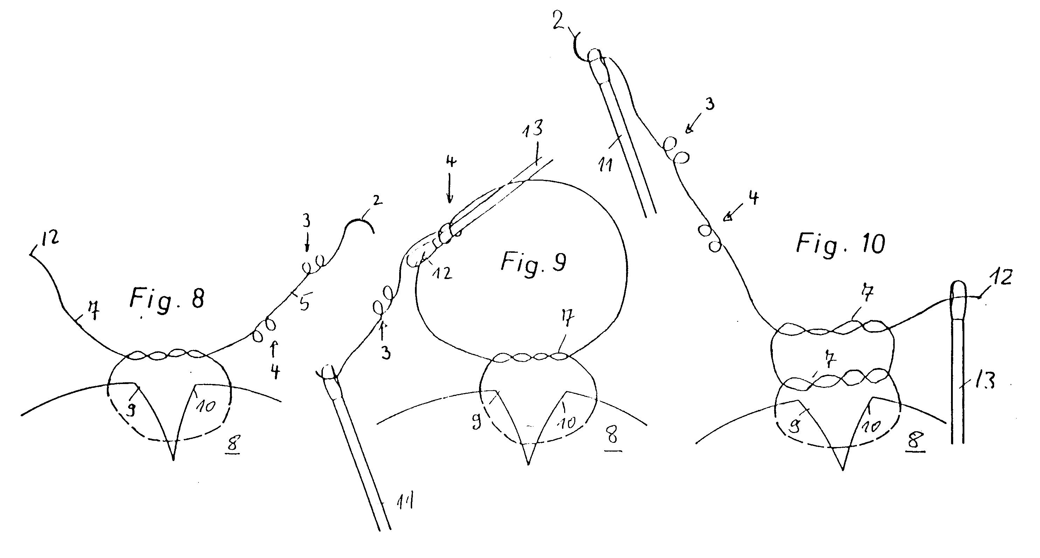

Der zum Sichern des chirurgischen Knotens erforderliche Endknoten wird dadurch gebildet, daß der oben beschriebene Vorgang mit den wendelförmigen Vorformungen des vorgeformten Teilbereiches 4 wiederholt wird, wie dies anhand der Figuren 8 bis 10 dargestellt ist. Fig.8 zeigt deutlich, wie der Grundknoten gebildet ist und nunmehr, zur Bildung des gegenläufigen Endknotens das freie Fadenende 12 durch die wendelförmigen Vorformungen des vorgeformten Teilbereiches 4 hindurchzuziehen ist.

Gemäß Fig.9 wird nun der zweite Nadelhalter 13 durch die wendelförmigen Vorformungen des vorgeformten Teilbereiches 4 hindurchgeführt, und zwar in Richtung des ersten vorgeformten Teilbereiches 3. Anschließend wird mit dem zweiten Nadelhalter 13 das freie Fadenende 12 ergriffen, dieses durch die wendelförmigen Vorformungen des Teilbereiches 4 zurückgezogen, wonach dann durch Auseinanderziehen des Endes 12 sowie des an der Nadel 2 angebrachten Fadenendes der chirurgische Knoten vollendet wird, wie dies anhand von Fig.10 angedeutet ist. Nachdem der Faden am Knoten abgeschnitten wurde, können in derselben Weise wie vorstehend beschrieben weitere Einzelknopfnähte mit diesem Faden 1 des Nahtmaterials durchgeführt werden.

Die wendelförmigen Vorformungen der Teilbereiche 3,4 sind aus einem unverformten Faden 1 durch plastische Formgebung gebildet, wobei die Formgebung der Gestalt ist, daß die wendelförmigen Vorformungen ohne Probleme durch das Gewebe 8 durchgezogen werden können und sich beim Durchziehen in eine etwa geradlinige Form strecken, so daß die Einstichkanäle nicht unnötig geweitet werden. Nach dem Durchziehen durch das Gewebe 8 kehrt der Faden 1 im Bereich der Teilbereiche 3,4 in seine vorgeformte Form zurück, so daß deren wendelförmigen Vorformungen wieder entstehen. Auch beim Zusammenziehen der Grund- und Endknoten eines chirurgischen Knotens tritt dieser Effekt auf. Die erforderliche Anzahl der wendelförmigen Gänge der vorgeformten Teilbereiche 3,4 ist abhängig von dem Knoten, den man damit erreichen möchte.

Grundsätzlich wird sich die in den Figuren 5 bis 10 dargestellte Vorgehensweise bei der Knotenbildung meist nur in schlecht zugänglichem oder nur unzureichend einsehbarem Operationsgebiet empfehlen bzw. nicht vermeiden lassen. Meist aber ist es ausreichend, den mit der Nadel 2 verbundenen, entsprechend bemessenen Abschnitt des Nahtmaterials durch das Gewebe 8 bis zu dem ersten vorgeformten Teilbereich 3 hindurchzuziehen und anschließend den Grund- und den Endknoten unter Verwendung des mit der Nadel 2 verbundenen Fadenendes zu legen.

Dabei ist es von besonderem Vorteil, lediglich das entsprechende Fadenende durch die Vorformungen des vorgeformten Teilbereiches 3,4 zu fädeln, anstatt mit den Zangenmaulteilen eines Nadel- oder Fadenhalters durch diese Windungen oder dergleichen durchzugreifen. Andernfalls muß nämlich das Nahtmaterial auf der gesamten Länge seines Fadens 1 - wie in den Zeichnungen dargestellt - durch das Gewebe 8 gezogen werden, einschließlich eines größeren Teils des unverformten Fadenabschnitts 7, da der Nadelhalter von einem ungünstigen Winkel, nämlich von der Seite des Wundgrundes her durch die Windungen oder dergleichen Vorformungen der vorgeformten Teilbereiche 3, 4 durchzuführen ist. Wenn das vorgeformte Fadenteil demgegenüber nicht durch die Wunde gezogen werden soll, muß der Nadelhalter ebenfalls von der Seite des Wundgrundes her durch die vorgeformten Teilbereiche 3, 4 durchgeführt werden. Beides kann eventuell die Handhabung des Nahtmaterials unter anderem durch den benötigten großen Raumbedarf zum Ausführen der Instrumentenbewegungen erschweren. Denn selbst bei günstigster Lage der vorgeformten Teilbereiche erfordert das Zuziehen des Knotens dann besonders viel Platz, wenn der Faden 1 mit zwei mehrgängigen, voneinander beabstandeten Wendeln mit einem entsprechend großen, an die Zangenmaulteile angepaßten Durchmesser ausgebildet ist. Ist dieser Raum zum Zuziehen des Knotens im Operationsfeld nicht gegeben, muß der Knoten schrittweise zugezogen werden, wobei eventuell auch mehrmals nachzufassen ist.

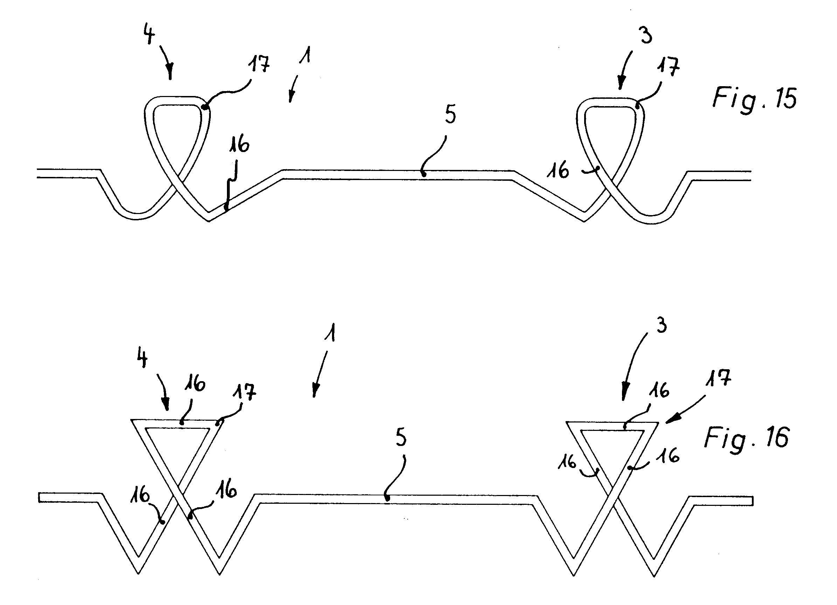

Ein bei geengtem Operationsfeld umständliches Durchführen der Zangenmaulteile von seiten des Wundgrundes her kann auch dadurch vermieden werden, daß die zu den Windungen oder dergleichen Vorformungen hin- und/oder wegführenden ungeformten Fadenteile in Richtung der angrenzenden vorgeformten Fadenabschnitte weggeführt sind, so daß die im Durchmesser an die Zangenmaulteile angepaßten Windungen oder dergleichen Vorformungen in ihrer etwa rechtwinklig zur Längserstreckung des Fadens liegenden Ebene um etwa 180° gekippt sind, wie dies beispielsweise in den Figuren 2 und 14 dargestellt ist.

Dabei ist der in Fig.2 dargestellte Faden 1 ähnlich dem Nahtmaterial aus Fig.1 ausgebildet, wobei die mit gegensinnigen Drehrichtungen wendelförmig vorgeformten Teilbereiche 3,4 durch einen in seiner Längserstreckung hier rechteckig abgewinkelten, aus geradlinigen Fadenstücken bestehenden Fadenabschnitt 5 voneinander getrennt sind und die von den Teilbereichen 3,4 abgehenden Fadenabschnitte 14,15 in entgegengesetzte Richtungen weisen, was dem Durchführen der Zangenmaulteile zum Fassen und Durchziehen des Fadenendes durch die Instrumentenposition des Faden- oder Nadelhalters entgegenkommen kann.

Ein solcher, entgegen der Längserstreckung des Fadens 1 verlaufender, vorgeformter Teilbereich kann den Raumbedarf der beispielsweise in den Figuren 5 bis 10 dargestellten Instrumentenbewegungen dadurch bereits vermindern, daß das Durchführen der Zangenmaulteile durch die Vorformungen jetzt in Richtung des Wundgrundes erfolgt, was der meist vertikalen Instrumentenstellung bei endoskopischen Operationen hilfreich entgegenkommt.

Dabei muß der vorgeformte Teilbereich des Fadens auch nicht mehr mit Hilfe eines zweiten Nadelhalters weit vom Wundgrund weggezogen werden, um die Zangenmaulteile des ersten Nadelhalters in richtiger Richtung durch die Vorformungen führen zu können, wie es beispielsweise in den Figuren 6 und 9 gut zu sehen ist.

Wird demgegenüber nicht der Nadelhalter durch die präformierten Schlingen oder dergleichen Vorformungen geführt, sondern lediglich das zu verknotende Fadenende, wird zum Knoten ein noch kleinerer Fadenabschnitt benötigt, so daß der Knoten auch unter beengten Platzverhältnissen durch Zuziehen eines vergleichsweise kurzen Fadenstückes gelegt werden kann. Das durch die beispielsweise wendel- oder spiralförmigen Vorformungen geschobene Fadenende wird dabei auf der anderen Seite gepackt und zur Knotenbildung zugezogen. Bei dieser bevorzugten Vorgehensweise müssen die eigenstabilen Windungen oder dergleichen Vorformungen in ihrem Durchmesser d nur geringfügig größer sein als der Durchmesser oder die Dicke des Fadenmaterials. Dabei erfolgt bei dieser bevorzugten Vorgehensweise die Durchführung des Fadens 1 nicht von der Seite des Wundgrundes aus, sondern von der dem Wundgrund abgewandten Seite. Das umständliche Durchführen der Zangenmaulteile bei beispielsweise ungünstigem Durchführungswinkel von Seiten des Wundgrundes her entfällt.

In den Figuren 3, 15 und 16 sind weitere Ausführungsformen des erfindungsgemäßen Nahtmaterials dargestellt, bei denen der Faden 1 jeweils ebenfalls zwei voneinander beabstandete, vorgeformte Teilbereiche mit entsprechenden Vorformungen aufweist, die eine Knotenbildung erfindungsgemäß auch unter beengten Platzverhältnissen erleichtern sollen.

Der Faden 1 des in Fig.3 dargestellten Nahtmaterials weist zwei spiralförmig vorgeformte Teilbereiche 3,4 mit gegensinnig gewundenen Spiralgängen auf, die voneinander beabstandet und brillenförmig zueinander angeordnet sind. Durch bloßes Durchfädeln eines Fadenendes durch das Zentrum 18 eines der beiden vorgeformten Teilbereiche 3,4 kann auf einfache Weise ein Grund- oder ein Endknoten eines chirurgischen Knotens gebildet werden.

In den Figuren 15 und 16 sind die etwa wendelförmigen Schlingen oder dergleichen Vorformungen der voneinander beabstandeten, vorgeformten Teilbereiche 3,4 - mehr oder weniger ausgeprägt - durch Geraden 16 und gekrümmte Linien oder Fadenabschnitte 17 gebildet. Dabei beschreibt jede Schlinge des in Fig.15 und 16 dargestellten Nahtmaterials praktisch eine Dreiecksform.

Möglich ist aber auch, die wendelförmigen Vorformungen der Teilbereiche 3,4 des Nahtmaterials jeweils als Polygon auszubilden oder auch nur einen vorgeformten Teilbereich im Nahtmaterial vorzusehen.

Wie aus Fig.15 deutlich wird, weisen die Teilbereiche 3,4 des dort dargestellten Fadens 1 die gleiche Drehrichtung auf. Zwei derart voneinander beabstandete, vorgeformte Teilbereiche mit gleichen Drehrichtungen können zweckmäßig sein, wenn die Windungen, Schlingen oder dergleichen Vorformungen der Teilbereiche 3,4 des Nahtmaterials durch Verklebungen oder dergleichen gebildet sind, die sich beim Durchziehen eines Fadenendes und Strecken des Nahtmaterials in diesem Teilbereich 3 oder 4 auflösen. Somit kann der Grundknoten beispielsweise mittels des ersten Teilbereiches 3 und der Endknoten mittels des weiteren vorgeformten Teilbereiches 4 gelegt werden.

Desweiteren ist ein Nahtmaterial, welches zwei voneinander beabstandete vorgeformte Teilbereiche mit gleichen Drehrichtungen aufweist, dort vorteilhaft einsetzbar, wo statt eines chirurgischen Knotens lediglich ein sogenannter Weiberknoten gelegt werden soll, bei dem ein Fadenteil zweimal um einen anderen Fadenteil geknotet ist. Ein solcher Weiberknoten kann auch dann noch nachgeben, wenn über den Grundknoten der sogenannte Endknoten gelegt ist. Ein solches Nachgeben des Knotens kann unter Inkaufnahme einer geringeren Knotensicherheit bei wenig zugbelastetem Gewebe dann wünschenswert sein, wenn zum Beispiel an kosmetisch wichtigen Stellen eine zu hohe Zugwirkung des Fadens und damit eine entsprechende Druckwirkung auf das Gewebe ausgeglichen werden soll, oder wenn bei empfindlichem Gewebe eine zu hohe Spannung infolge des postoperativen Wundödems vermieden werden soll, um Minderdurchblutung, verzögerte Wundheilung und ungünstige Vernarbungen zu vermeiden.

In Fig.11 ist ein chirurgisches Nahtmaterial dargestellt, dessen Faden 1 in einem Teilbereich 3 seiner Längserstreckung vier ineinander übergehende, etwa kreisförmig vorgeformte Windungen 19 aufweist, durch die zur Schlaufen- und/oder Knotenbildung eines der beiden Fadenenden 26,27 des Fadens 1 durchführbar ist. Ähnlich wie in Fig.1, kann auch der Faden des in Fig.11 dargestellten Nahtmaterials an zumindest einem seiner Fadenenden mit einer Nadel verbunden sein.

Ebenso wie in Fig.4 weisen auch die beiden jeweils benachbarten Windungen 19 des in Fig.11 dargestellten Nahtmaterials gegensinnige Drehrichtungen auf, wobei die Zentralachsen 20,21 dieser benachbarten gegensinnigen Windungen 19 voneinander beabstandet sind. Jeweils zwei einander benachbarte Windungen 19 mit gegensinnigen Drehrichtungen sind in etwa einer Ebene angeordnet und bilden zusammen eine etwa acht-förmige Doppelschlaufe 22. Dabei ist, in derselben Fadenrichtung R1 betrachtet, der Einlaufabschnitt 23 der einen Windung 19a und der Auslaufabschnitt 24 der anderen Windung 19b auf gegenüberliegenden Seiten ihres gemeinsamen Übergangsbereiches 25 angeordnet.

Der in Fig.11 dargestellte vorgeformte Teilbereich 3 weist lediglich zwei Doppelschlaufen 22 auf, so daß jeder Windung 19 des vorgeformten Teilbereiches 3 mit der einen Drehrichtung eine weitere Windung 19 mit entgegengesetzter Drehrichtung zugeordnet ist, was einer unerwünschten Verdrehung des Fadens entgegenwirkt. Durch die etwa acht-förmige Ausgestaltung der beiden vorgeformten Doppelschlaufen 22 haben die jeweils einander benachbarten Windungen gegensinnigen Drehrichtungen, wobei die Zentralachsen 20,21 der Windungen 19 mit gleicher Drehrichtung etwa koaxial zueinander angeordnet sind. Durch die koaxial zueinander angeordneten Windungen 19 mit gleicher Drehrichtung kann mit geringem Aufwand das entsprechende Fadenende 26,27 durchgezogen und ein Grund- oder ein Endknoten gelegt werden. So wird beispielsweise das in Fig.11 rechte Fadenende 26 auf der gegenüberliegenden Seite des vorgeformten Teilbereiches 3 beispielsweise in die oberen, eine gleiche Drehrichtung aufweisenden Windungen 19 eingeführt und durch diese durchgezogen, so daß ein doppelt geschlungener, sogenannter chirurgischer Grundknoten entsteht. Sobald der Grundknoten gelegt ist und der Faden entlastet wird, bildet sich der in Fig.11 dargestellte vorgeformte Teilbereich aus elastischem Material erneut, so daß das Fadenende zum Legen des Endknotens nun durch die unteren Windungen 19 mit - im Vergleich zu den oberen Windungen - gegensätzlicher Drehrichtung geführt und durchgezogen werden kann. Es entsteht ein sogenannter Schifferknoten, dessen in entgegengesetzte Richtungen geschlungenen Knoten bekanntermaßen einen besonders sicheren Knotensitz gewährleisten.

Soll der Endknoten dagegen nur einfach geschlungen werden, so wird das Fadenende 26 nur durch eine der beiden unteren Windungen 19 geführt.

Um beispielsweise den Grundknoten als doppelt geschlungenen chirurgischen Knoten ausbilden zu können und um anschließend den Endknoten nur einfach geschlungen auszuführen, ist auch eine Ausführungsform ausreichend, bei der der vorgeformte Teilbereich des Nahtmaterials drei ineinander übergehende Windungen 19 hat, wobei zwei Windungen 19 mit gleicher Drehrichtung eine Windung 19 mit dazu gegensinniger Drehrichtung zwischen sich einschließen.

Erfordern die Umstände demgegenüber einen nachgiebigen Sitz in Form eines sogenannten Frauen- oder Weiberknotens, so ist das entsprechende Fadenende 26 zum Legen des Grundknotens sowie des Endknotens durch dieselben Windungen 19 in Pfeilrichtung R2 zu ziehen.

In Fig.12 ist ein chirurgisches Nahtmaterial dargestellt, dessen Faden 1 drei Doppelschlaufen 22 aufweist. Diese drei Doppelschlaufen 22 werden durch sechs ineinander übergehende, kreisförmig vorgeformte Windungen, Schlaufen oder dergleichen Vorformungen 19 gebildet, wobei die einander benachbarten Vorformungen jeweils gegensinnige Drehrichtungen haben. Jeweils zwei benachbarte Windungen 19 mit gegensätzlichen Drehrichtungen sind in etwa einer Ebene angeordnet und bilden eine der etwa acht-förmig vorgeformten Doppelschlaufen 22. Auch hier sind die Zentralachsen 20,21 der jeweils drei Windungen 19 mit gleicher Drehrichtung etwa koaxial zueinander angeordnet. Der in Fig.12 dargestellte vorgeformte Teilbereich 3 des Fadens 1 bildet eine Vielzahl von Möglichkeiten zur Knotenbildung. Wird beispielsweise ein Fadenende 26,27 auf der gegenüberliegenden Seite durch alle drei Windungen mit gleicher Drehrichtung gezogen, so bildet sich ein dreifach geschlungener, vergleichsweise sicherer Grundknoten. Die einzelnen Doppelschlaufen 22 sind geringfügig voneinander beabstandet, so daß zur Knotenbildung auf einfache Weise auch nur eine Windung 19 oder zwei Windungen 19 mit gleicher Drehrichtung verwendet werden können.

Zweckmäßigerweise weist die Windung 19 oder die Windungen 19 zumindest mit der einen Drehrichtung einen lichten Durchmesser d auf, der - zum Durchfädeln eines Fadenendes durch diese Vorformungen 19 - zumindest der Dicke des durchzuführenden Fadens oder Fadenendes entspricht. Auch wenn die andere Windung 19 mit dazu entgegengesetzter Drehrichtung einen kleineren Durchmesser hat, durch das eines der beiden Enden des Nahtmaterials nur schwer durchzuführen ist, so wird doch einer unerwünschten Verdrehung des chirurgischen Nahtmaterials und insbesondere seines Fadens 1 bei der Knotenbildung entgegengewirkt. Zweckmäßig ist es, wenn alle Windungen 19 des vorgeformten Teilbereiches 3 - wie hier dargestellt - einen Durchmesser d aufweisen, durch den die Zangenmaulteile eines Nadel- oder Fadenhalters durchführbar sind, so daß der Fadenhalter durch die Windungen 19 hindurchgreifen und zur Knotenbildung das entsprechende Fadenende 26,27 erfassen kann.

Möglich ist auch, den Faden beispielsweise mit drei ineinander übergehenden kreisförmig vorgeformten Windungen 19 auszugestalten, wobei die Windungen 19 in etwa einer Ebene angeordnet sind und benachbarte Windungen 19 gegensätzliche Drehrichtungen aufweisen. Auch kann nach zwei Windungen 19 mit gegensätzlichen Drehrichtungen das Nahtmaterial zunächst in eine Windung 19 übergehen, die dieselbe Drehrichtung wie zumindest die auf einer Seite zu ihr benachbarte Windung 19 aufweist. Bereits derartige Ausführungsformen wirken einer unerwünschten Verdrehung des erfindungsgemäßen Nahtmaterials und insbesondere seines Fadens 1 entgegen, können jedoch eine solche Verdrehung nicht vollständig verhindern, weil nicht jeder Windung 19 des vorgeformten Teilbereiches 3 mit der einen Drehrichtung eine weitere Windung 19 mit entgegengesetzter Drehrichtung zugeordnet ist.

In den Figuren 11 bis 14 sind beide der an die vorgeformten Windungen 19 angrenzenden Fadenabschnitte 14,15 rechtwinklig zu der parallel zu den benachbarten Windungen 19 und den Doppelschlaufen 22 verlaufenden Ebene abgewinkelt. Dabei gehen in den Figuren 11 und 12 die äußeren Windungen 19 unmittelbar in den abgewinkelten Fadenabschnitt 14,15 über, während in den Figuren 13,14 die äußeren Windungen 19 zunächst jeweils in einer halbkreisförmige Windung oder Krümmung 28 mit entgegengesetzter Drehrichtung übergehen, an die sich dann auf der dem zwischen der Windung 19 und der Krümmung 28 vorgesehenen Übergangsbereich 29 abgewandten Seite der Krümmung 28 der etwa rechtwinklig abgewinkelte Fadenabschnitt 14,15 anschließt.

Die in den Figuren 13 und 14 dargestellten Teilbereiche 3 gestatten eine schnellere Lagebestimmung der Windungen, Schlingen oder dergleichen Vorformungen 19 und der daran angrenzenden Fadenenden 26 und 27, was einer einfachen Handhabung des Fadens und des Nahtmaterials insgesamt zusätzlich entgegenkommt.

Dabei sind die in Fig.14 an die vorgeformten Windungen 19 angrenzenden Fadenabschnitte 14,15 außerhalb der Windungen 19 etwa rechtwinklig zu dem angrenzenden vorgeformten Teilbereich 3 abgewinkelt, was das Durchführen eines Fadenendes 26,27 auf der gegenüberliegenden Seite durch die entsprechenden Windungen 19 wesentlich vereinfachen kann und insbesondere erlaubt, beispielsweise auch einen Nadel- oder Fadenhalter von der dem Knotensitz abgewandten Seite durch den vorgeformten Teilbereich des Nahtmaterials zu führen, um das andere Fadenende durchzuziehen, was der vertikalen Instrumentenstellung bei endoskopischen Operationen hilfreich entgegenkommen und den Raumbedarf der Instrumentenbewegungen verringern kann. Im übrigen sind auch die in den Figuren 13 und 14 dargestellten Fäden, ähnlich wie in Fig.11, mit zwei etwa acht-förmig vorgeformten Doppelschlaufen 22 ausgestaltet.

In Fig.4 ist demgegenüber der Faden 1 eines chirurgischen Nahtmaterials dargestellt, bei dem die Windungen, Schlingen oder dergleichen Vorformungen 19 des vorgeformten Teilbereiches 3 sowie die angrenzenden Fadenabschnitte 14,15 praktisch in etwa einer Ebene angeordnet sind. Dieses chirurgische Nahtmaterial ist daher vergleichsweise flach ausgestaltet. Auch bei dem in Fig.4 dargestellten Nahtmaterial ist auf beiden Seiten des vorgeformten Teilbereiches 3 jeweils eine Krümmung 28 vorgesehen, die eine zur benachbarten Windung, Schlinge oder dergleichen Vorformung 19 entgegengesetzte Drehrichtung aufweist. Da der vorgeformte Teilbereich 3 des in Fig.4 dargestellten Nahtmaterials im wesentlichen aus zwei Doppelschlaufen 22 gebildet ist, an die sich auf der einen Seite eine weitere kreisförmige Windung oder Schlinge 19 anschließt, sind die an den vorgeformten Teilbereich 3 angrenzenden Fadenabschnitte 14,15 auf derselben Längsseite dieses Teilbereiches 3 angeordnet.

Wenn der vorgeformte Teilbereich des erfindungsgemäßen Nahtmaterials aus elastischem Material besteht, das ohne Einwirkung einer Zugbelastung in seine ursprüngliche, etwa acht-förmig oder dergleichen vorgeformte Formgebung zurückfällt, kann das Nahtmaterial zunächst auch, selbst wenn der vorgeformte Teilbereich in seinem Durchmesser größer als der Innendurchmesser einer Trokarhülse ist, durch diese Trokarhülse oder das zu versorgende Gewebe gezogen werden, um anschließend dennoch zumindest bereichsweise die zum Knoten gewünschte acht- oder brillenförmige oder dergleichen vorgeformte Formgebung anzunehmen. Das in den Figuren 1 bis 16 in verschiedenen Ausführungsformen dargestellte chirurgische Nahtmaterial ist daher nicht nur bei Eingriffen am eröffneten Abdomen sowie bei Hautnähten einsetzbar, sondern insbesondere auch bei endoskopischen intrakorporalen Operationen.

Auch die Windungen, Schlingen oder dergleichen Vorformungen 19 der in den Figuren 4 sowie 11 bis 14 dargestellten Nahtmaterialien können in ihren Teilbereichen 3 durch Verkleben oder dergleichen vorgeformt werden. Um auch bei einer derartigen Ausführungsform einen Doppelknoten mit einem Grundknoten sowie einem Endknoten legen zu können, kann es zweckmäßig sein, wenn auch die in den Figuren 4 sowie 11 bis 14 dargestellten Nahtmaterialien zwei voneinander beabstandete vorgeformte Teilbereiche mit acht-förmigen Doppelschlaufen 22 aufweisen.

Durch die etwa rechtwinklig abgewinkelte Position der in den Figuren 1, 2 und 5 bis 16 an die vorgeformten Teilbereiche 3,4 angrenzenden Fadenabschnitte 5, 14 und 15 bilden die Windungen, Schlingen oder dergleichen Vorformungen 19 dieser vorgeformten Teilbereiche 3,4 praktisch eine quer zur Längserstreckung des Nahtmaterials angeordnete Fadenöffnung, durch die auf einfache Weise die beiden Zangenmaulteile eines üblichen Faden- oder Nadelhalters oder das durch die Windungen durchzuführende Fadenende des Fadens durchgeführt werden können. Dadurch wird das Anlegen von Knoten auch unter beengten Raumverhältnisses wesentlich erleichtert. Um die Drehrichtung der entsprechenden Windungen des vorgeformten Teilbereiches gut erkennen und auch bei einem geradlinig gestreckten Nahtmaterial die Position dieses vorgeformten Teilbereiches 3 und/oder 4 abschätzen zu können, ist es zweckmäßig, wenn die Windungen oder dergleichen Vorformungen zumindest mit der einen Drehrichtung eine, Signaleinfärbung, Signalbeschichtung oder dergleichen Signalmarkierung aufweisen.

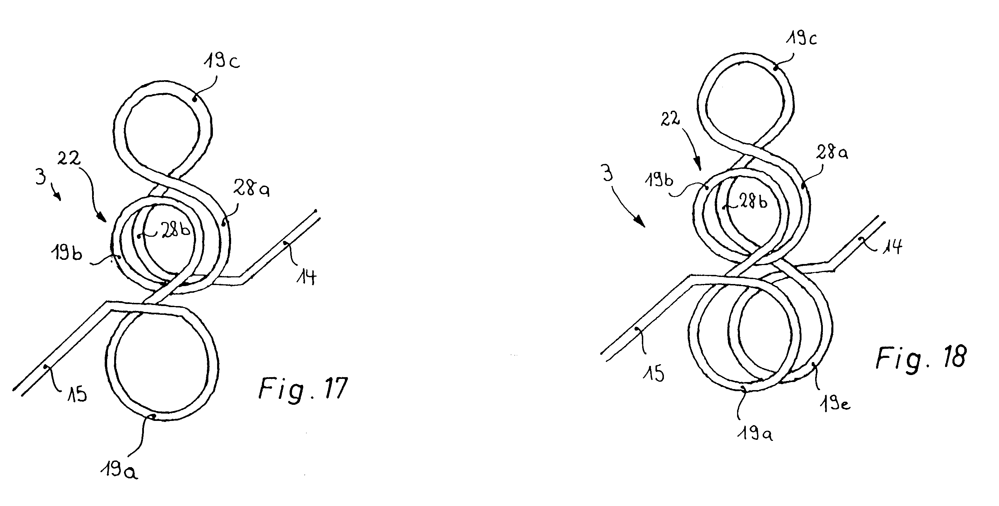

In den Figuren 17 und 18 sind zwei vorgeformte Teilbereiche 3 eines chirurgischen Nahtmaterials dargestellt, die sich nur geringfügig voneinander unterscheiden, jedoch für unterschiedliche Anwendungszwecke vorgesehen sind.
Dabei weisen die in den Figuren 17 und 18 dargestellten, vorgeformten Teilbereiche 3 jeweils eine etwa acht-förmige Doppelschlaufe 22 auf, die aus einer ersten Windung, Schlinge oder dergleichen Vorformung 19a sowie einer zweiten Vorformung 19b mit dazu gegensinniger Drehrichtung gebildet ist. Diese zweite Vorformung 19b der Doppelschlaufe 22 geht in eine erste Krümmung 28a mit gleichsinniger Drehrichtung über, wobei sich an diese erste Krümmung 28a auf ihrer der zweiten Vorformung 19b abgewandten Seite eine dritte Windung, Schlinge oder dergleichen Vorformung 19c mit einer zur ersten Krümmung 28a gegensinnigen Drehrichtung anschließt. Diese dritte Vorformung 19c geht ihrerseits in eine zweite Krümmung 28b mit zu ihr gegensinniger Drehrichtung über, wobei die kreisbogenförmigen Krümmungen 28a, 28b zusammen den Umriß einer vierten Schlinge bilden, deren Schlingenöffnung sich mit der Schlingenöffnung der zweiten Vorformung 19b praktisch überdeckt. Dabei sind die Zentralachsen der ersten, zweiten und dritten Vorformung 19a, 19b und 19c voneinander beabstandet, achsparallel zueinander angeordnet und verlaufen in praktisch einer Ebene. Die Zentralachse der vierten, durch die Krümmungen 28a, 28b gebildeten Vorformung ist etwa koaxial zur Zentralachse der zweiten Vorformung 19b angeordnet.

Während bei dem in Figur 17 dargestellten Teilbereich 3 der von der zweiten Krümmung 28b abgehende Fadenabschnitt 14 praktisch rechtwinklig abgewinkelt ist und ebenfalls etwa achsparallel zu den Zentralachsen der Vorformungen 19 verläuft, schließt sich bei dem in Figur 18 dargestellten Teilbereich 3 an die zweite Krümmung 28b eine fünfte Windung, Schlinge oder dergleichen Vorformung 19e mit einer zur Krümmung 28b gegensinnigen Drehrichtung an. Diese Schlinge 19e, deren Zentralachse etwa koaxial zur Zentralachse der ersten Vorformung 19a angeordnet ist, bildet mit der durch die Krümmungen 28a, 28b gebildeten vierten Vorformung sowie mit der dritten Vorformung 19c einen aus drei Schlingen gebildeten Fadenabschnitt.

Dabei kann bei dem gemäß Figur 17 vorgeformten Teilbereich 3 auf die oben beschriebene Signalmarkierung verzichtet werden. Denn der vorgeformte Teilbereich 3 gemäß Figur 17 gestattet auf einfache Weise beispielsweise das Legen eines chirurgischen Grundknotens mit abschließendem einfachen Endknoten als Schifferknoten, indem beispielsweise das Fadenende für den Grundknoten durch die mittlere Öffnung der durch die Krümmungen 28a, 28b gebildeten Vorformung sowie die Windung 19b und anschließend für den Endknoten durch eine der beiden äußeren Öffnungen der Vorformungen 19a, 19c geführt wird. Da sich eine sichere Orientierung aufgrund der Position der drei beabstandeten Öffnungen zueinander im vorgeformten Teilbereich 3 von selbst ergibt, kann bei der in Figur 17 dargestellten Ausführungsform auf eine Signalmarkierung der Windungen verzichtet werden.

Demgegenüber kann mit dem in Figur 18 dargestellten Teilbereich 3 ein sicherer, fester Knoten auch bei solch steifem Nahtmaterial gebildet werden, das eine besonders glatte Oberfläche und dadurch bedingt eine nur geringe Haftreibung im Bereich des Knotensitzes aufweist. Mit einem gemäß Figur 18 ausgebildeten Teilbereich 3 des Nahtmaterials läßt sich dann beispielsweise leicht ein chirurgischer Knoten legen, gesichert mit einem zweiten, chirurgisch gelegten Knoten als Schifferknoten, wobei dieser zweite Knoten dann seinerseits nochmal durch einen weiteren einfachen Knoten als Schifferknoten gesichert wird. Ein solch doppelt gesicherter chirurgischer Knoten wird oft auch bei Operationen am eröffnetem Abdomen und besonders bei Verwendung der oben erwähnten Fäden mit besonders glatter Oberfläche eingesetzt und ist im Rahmen einer endoskopisch intrakorporalen Operation mit dem bisher zur Verfügung stehenden Fadenmaterial nur mit viel Geschick und unter erheblichem zeitlichem Aufwand erreichbar.

Wie die Figuren 17 und 18 zeigen, sind die durch die Schlingen 19 oder Krümmungen 28 gebildeten Übergangsbereiche in ihren Kreuzungsabschnitten im Verlauf der Längserstreckung des Fadens oder Nahtmaterials nacheinander regelmäßig in eine Richtung versetzt zueinander angeordnet.

Nach einer hier nicht dargestellten Ausführungsform gemäß der Erfindung ist vorgesehen, das Nahtmaterial zwei- oder mehrteilig auszugestalten, wobei der vorgeformte Teilbereich oder die vorgeformten Teilbereiche 3,4 sowie der dazwischenliegende Fadenabschnitt 5 beispielsweise aus elastischem Material den einen Teil und zumindest einer der beiden an die Teilbereiche 3,4 angrenzenden Fadenabschnitte 14,15 jeweils einen anderen Teil dieses mehrteiligen Nahtmaterials bilden. Dabei können die vorgeformten Teilbereiche 3,4 und die angrenzenden, gegebenenfalls auch aus unelastischem Material bestehenden Fadenabschnitte mittels eines Steck-, Klemm- oder dergleichen Verbindungselementes gegebenenfalls lösbar miteinander verbunden sein. Insbesondere ein mit dem Faden 1 des erfindungsgemäßen Nahtmaterials lösbar verbindbares Verbindungselement erlaubt es, die vorgeformten Teilbereiche mehrmals zu verwenden und auch mit solchem Fadenmaterial zu verbinden, das - beispielsweise im Gegensatz zu den vorgeformten und nach der Knotenbildung zu entfernenden Teilbereichen 3,4 des Nahtmaterials, aus einem besonders gewebeverträglichen und gegebenenfalls unelastischen Material besteht. Möglich ist auch, den Faden und/oder jeweils zumindest den vorgeformten Teilbereich 3,4 des Nahtmaterials aus einem memory-Metall, insbesondere aus einer Nickel-Titan-Metallegierung, herzustellen, die beispielsweise temperaturabhängig von ihrer gestreckten, leicht durch eine Trokarhülse zu führenden Form in ihre bereichsweise vorgeformte Gestalt fällt.

Eine Knotenbildung ist zwar bei jeweils eingängigen Wendeln oder Spiralen oder auch bei nur einer Doppelschlaufe möglich, in der Praxis werden jedoch mehrgängige Wendeln oder Spiralen oder mehrere Doppelschlaufen bevorzugt, um die Haltewirkung und Festigkeit des mittels des erfindungsgemäßen Nahtmaterials gelegten Knotens sicherzustellen.

Das Nahtmaterial kann als Meterware beispielsweise mit sich in ihrer Drehrichtung abwechselnden vorgeformten Teilbereichen 3,4 und dazwischenliegenden ungeformten Fadenabschnitten 5 gebildet sein, was besonders kostengünstig in der Herstellung ist. Möglich ist aber auch, das Nahtmaterial mit nur einem Faden 1 sowie einer damit verbundenen Nadel 2 zu konfektionieren, was einer schnellen und sicheren Handhabung zugute kommt und auch die Sterilität des Nahtmaterials gewährleistet.

## Patentansprüche

1. Chirurgisches Nahtmaterial mit einem Faden (1) und gegebenenfalls einer Nadel (2) an zumindest einem Fadenende, wobei das Nahtmaterial in zumindest einem ersten vorgeformten Teilbereich (3,4) seiner Längserstreckung wenigstens eine eigenstabile Windung, Schlinge oder dergleichen Vorformung aufweist, **dadurch gekennzeichnet**, daß der vorgeformte Teilbereich (3,4) als etwa wendel- oder spiralförmige Vorformung ausgebildet ist, durch die ein Teilbereich des Fadens oder ein Fadenende zur Schlaufen- und/oder Knotenbildung durchfädelbar oder durchführbar ist und daß das Nahtmaterial zumindest zwei voneinander beabstandete vorgeformte Teilbereiche (3,4) hat, deren wendel- oder spiralförmigen Vorformungen gegensinnige Drehrichtungen aufweisen.

2. Chirurgisches Nahtmaterial nach Anspruch 1, dadurch gekennzeichnet, daß die wendel- oder spiralförmige Vorformung zumindest eines Teilbereiches (3,4), vorzugsweise von zwei Teilbereichen (3,4) des Nahtmaterials jeweils mehrere, zweckmäßigerweise zwei Windungen aufweist.

3. Chirurgisches Nahtmaterial nach Anspruch 1 oder 2, dadurch gekennzeichnet, daß jeder wendel- oder spiralförmig vorgeformte Teilbereich (3,4) des Nahtmaterials zum Durchfädeln eines Fadenendes geringfügig größer als der Durchmesser des Fadenmaterials und gegebenenfalls zum Durchführen eines chirurgischen Nadel- oder Fadenhalters in seinem lichten Durchmesser (d) an die Außenabmessungen dieses Fadenhalters im Bereich von dessen Zangenmaulteile angepaßt ist.

4. Chirurgisches Nahtmaterial nach dem Oberbegriff von Anspruch 1, dadurch gekennzeichnet, daß das Nahtmaterial zumindest in seinem ersten vorgeformten Teilbereich (3) seiner Längserstreckung wenigstens zwei ineinander übergehende Windungen, Schlingen oder dergleichen Vorformungen (19) aufweist, welche gegensinnige Drehrichtungen haben, daß durch mindestens eine dieser Windungen oder dergleichen Vorformungen (19) ein weiterer Teilbereich des Fadens oder ein Fadenende (26,27) zur Schlaufen- und/oder Knotenbildung durchführbar ist und daß die Zentralachsen (20,21) dieser benachbarten gegensinnigen Windungen oder dergleichen Vorformungen (19) voneinander beabstandet sind.

5. Chirurgisches Nahtmaterial nach Anspruch 4, dadurch gekennzeichnet, daß jeweils zwei einander benachbarte Windungen oder dergleichen Vorformungen (19) mit gegensinnigen Drehrichtungen in etwa einer Ebene angeordnet sind.

6. Chirurgisches Nahtmaterial nach Anspruch 4, dadurch gekennzeichnet, daß jeweils zwei benachbarte Windungen oder dergleichen Vorformungen (19) mit gegensinnigen Drehrichtungen eine etwa acht-förmige Vorformung oder Doppelschlaufe (22) bilden.

7. Chirurgisches Nahtmaterial nach Anspruch 6, dadurch gekennzeichnet, daß das Nahtmaterlal zwei voneinander beabstandete vorgeformte Teilbereiche (3,4) hat, deren acht-förmigen Vorformungen oder Doppelschlaufen (22) insbesondere gegensinnige Drehrichtungen aufweisen.

8. Chirurgisches Nahtmaterial nach einem der Ansprüche 4 bis 7, dadurch gekennzeichnet, daß von zwei einander benachbarten Windungen oder dergleichen Vorformungen (19) mit gegensinnigen Drehrichtungen der Einlaufabschnitt (23) der einen Windung (19a) und der Auslaufabschnitt (24) der anderen Windung (19b) auf gegenüberliegenden Seiten ihres gemeinsamen Übergangsbereiches (25) angeordnet sind.

9. Chirurgisches Nahtmaterial nach einem der Ansprüche 4 bis 8, dadurch gekennzeichnet, daß jeder Windung oder dergleichen Vorformung (19) des vorgeformten Teilbereiches (3) mit der einen Drehrichtung eine weitere Windung (19) mit entgegengesetzter Drehrichtung zugeordnet ist.

10. Chirurgisches Nahtmaterial nach einem der Ansprüche 4 bis 9, dadurch gekennzeichnet, daß der vorgeformte Teilbereich (3) des Nahtmaterials mehr als zwei ineinander übergehende Windungen oder dergleichen Vorformungen (19) aufweist, daß jeweils benachbarte Vorformungen (19) gegensinnige Drehrichtungen haben und daß die Zentralachsen (20,21) der Vorformungen (19) mit gleicher Drehrichtung etwa koaxial zueinander angeordnet sind.

11. Chirurgisches Nahtmaterial nach einem der Ansprüche 4 bis 10, dadurch gekennzeichnet, daß der vorgeformte Teilbereich (3) des Nahtmaterials drei ineinander übergehende Windungen oder dergleichen Vorformungen (19) hat, wobei zwei Vorformungen (19) mit gleicher Drehrichtung eine Vorformung (19) mit dazu gegensinniger Drehrichtung zwischen sich einschließen.

12. Chirurgisches Nahtmaterial nach einem der Ansprüche 4 bis 11, dadurch gekennzeichnet, daß zumindest eine, vorzugsweise beide der äußeren Windungen oder dergleichen Vorformungen (19) des vorgeformten Teilbereiches (3) jeweils in eine halbkreisförmige Krümmung (28) mit entgegengesetzter Drehrichtung übergehen und daß im Anschluß daran der an den vorgeformten Teilbereich (3) jeweils angrenzende Abschnitt (14,15) des Nahtmaterials auf der dem zwischen der Windung oder dergleichen Vorformung (19) und der Krümmung (28) vorgesehenen Übergangsbereich (29) abgewandten Seite der Krümmung (28) abgewinkelt ist.

13. Chirurgisches Nahtmaterial nach einem der Ansprüche 4 bis 12, dadurch gekennzeichnet, daß zumindest einer, vorzugsweise beide der an den vorgeformten Teilbereich (3) angrenzenden Abschnitte (14,15) des Nahtmaterials quer, insbesondere rechtwinklig zu der durch die benachbarten Windungen oder dergleichen Vorformungen und/oder die Doppelschlaufen (22) gebildeten Ebene abgewinkelt sind.

14. Chirurgisches Nahtmaterial nach einem der Ansprüche 4 bis 13, dadurch gekennzeichnet, daß der Abschnitt(die Abschnitte) (14,15) des Nahtmaterials außerhalb der Windungen oder dergleichen Vorformungen in Richtung zu dem angrenzenden vorgeformten Teilbereich (3,4) abgewinkelt sind.

15. Chirurgisches Nahtmaterial nach dem Oberbegriff von Anspruch 1, dadurch gekennzeichnet, daß der vorgeformte Teilbereich (3) eine etwa 8-förmige Doppelschlaufe (22) aufweist, die aus einer ersten Windung, Schlinge oder dergleichen Vorformung (19a) sowie einer zweiten Vorformung (19b) mit dazu gegensinniger Drehrichtung gebildet ist, daß die zweite Vorformung (19b) dieser Doppelschlaufe (22) in eine erste Krümmung (28a) mit gleichsinniger Drehrichtung übergeht, daß die erste Krümmung (28a) in dem der ersten Vorformung (19a) abgewandten Bereich der Doppelschlaufe (22) in eine dritte Windung, Schlinge oder dergleichen Vorformung (19c) mit einer zur ersten Krümmung (28a) gegensinnigen Drehrichtung übergeht, daß sich an die dritte Vorformung (19c) eine zweite Krümmung (28b) mit zu ihr gegensinniger Drehrichtung anschließt, daß die kreisbogenförmigen Krümmungen (28a, 28b) den Umriß einer vierten Schlinge bilden, deren Schlingenöffnung sich zumindest bereichsweise mit der Schlingenöffnung der zweiten Vorformung (19b) überschneidet und daß durch diese Vorformungen (19) und Schlingenöffnungen ein Teilbereich des Fadens oder ein Fadenende zur Schlaufen- und/oder Knotenbildung durchfädelbar oder durchführbar ist.

16. Chirurgisches Nahtmaterial nach Anspruch 15, dadurch gekennzeichnet, daß sich an die zweite Krümmung (28b) eine fünfte Windung, Schlinge oder dergleichen Vorformung (19e) mit einer zu ihr gegensinnigen Drehrichtung anschließt.

17. Chirurgisches Nahtmaterial nach Anspruch 15 oder 16, dadurch gekennzeichnet, daß die Zentralachsen der Windungen, Schlingen oder dergleichen Vorformungen (19) etwa achsparallel zueinander und vorzugsweise in einer Ebene verlaufen und daß die Zentralachsen der zweiten und der vierten sowie gegebenenfalls auch der ersten und fünften Vorformung etwa koaxial zueinander angeordnet sind.

18. Chirurgisches Nahtmaterial nach einem der Ansprüche 1 bis 17, dadurch gekennzeichnet, daß die Windungen, Schlingen oder dergleichen Vorformungen und/oder durch Krümmungen gebildeten Übergangsbereiche in ihren Kreuzungsabschnitten im Verlauf der Längserstreckung des Nahtmaterials nacheinander regelmäßig in eine Richtung versetzt zueinander angeordnet sind.

19. Chirurgisches Nahtmaterial nach einem der Ansprüche 1 bis 18, dadurch gekennzeichnet, daß die Längs- oder Zentralachse(n) (1,20,21) des vorgeformten Teilbereiches (der vorgeformten Teilbereiche) (3,4) in Richtung der Längserstreckung des Nahtmaterials angeordnet ist(sind).

20. Chirurgisches Nahtmaterial nach dem Oberbegriff von Anspruch 1, dadurch gekennzeichnet, daß das Nahtmaterial zwei einander benachbarte Windungen oder dergleichen Vorformungen (19) oder zwei benachbarte vorgeformte Teilbereiche (3,4) aufweist, die mit gegensinnigen Drehrichtungen brillenförmig zueinander angeordnet sind und daß durch diese Vorformungen oder vorgeformten Teilbereiche ein weiterer Teilbereich des Fadens oder ein Fadenende durchführbar ist.

21. Chirurgisches Nahtmaterial nach Anspruch 20, dadurch gekennzeichnet, daß der Einlaufabschnitt der einen Windung oder dergleichen Vorformung und der Auslaufabschnitt der anderen Windung auf derselben Seite ihres gemeinsamen Übergangsbereiches angeordnet sind.

22. Chirurgisches Nahtmaterial nach einem der Ansprüche 1 bis 21, dadurch gekennzeichnet, daß die vorgeformten Teilbereiche des Nahtmaterials, insbesondere die Windungen oder dergleichen Vorformungen mit derselben Drehrichtung und/oder die zwischen zwei benachbarten vorgeformten Teilbereichen vorgesehenen Abschnitte (5) und/oder die zu den Vorformungen hin- und/oder wegführenden Abschnitte des Nahtmaterials eine Signaleinfärbung, Signalbeschichtung oder dergleichen Signalmarkierung aufweisen.

23. Chirurgisches Nahtmaterial nach einem der Ansprüche 1 bis 22, dadurch gekennzeichnet, daß zumindest sein vorgeformter Teilbereich (3,4) aus elastischem Material besteht.

24. Chirurgisches Nahtmaterial nach einem der Ansprüche 1-3, 7 bis 14 und 20 bis 23 dadurch gekennzeichnet, daß zwischen den voneinander beabstandeten vorgeformten Teilbereichen (3,4) ein unverformter Abschnitt (5) des Nahtmaterials angeordnet ist.

25. Chirurgisches Nahtmaterial nach einem der Ansprüche 1 bis 24, dadurch gekennzeichnet, daß sich an den vorgeformten Teilbereich (die vorgeformten Teilbereiche) (3,4) vorzugsweise beidseits ein unverformter Abschnitt (14,15) des Nahtmaterials anschließt und daß diese Abschnitte (14,15) vom vorgeformten Teilbereich (von den vorgeformten Teilbereichen) (3,4) wegweisen.

26. Chirurgisches Nahtmaterial nach einem der Ansprüche 1 bis 25, dadurch gekennzeichnet, daß der vorgeformte Teilbereich (die vorgeformten Teilbereiche) des Nahtmaterials als Teilstück(e) ausgebildet ist (sind), welche(s) mit dem(den) angrenzenden Faden- oder Nahtmaterial-Abschnitt(en) (5, 14, 15) mittels eines Steck-, Klemm- oder dergleichen Verbindungselementes lösbar verbunden ist.

27. Chirurgisches Nahtmaterial nach einem der Ansprüche 1 bis 26, dadurch gekennzeichnet, daß der Faden (1) und/oder der vorgeformte Teilbereich (die vorgeformten Teilbereiche) (3,4) des Nahtmaterials aus einem memory-Metall, insbesondere aus einer Nickel-Titan-Metall-Legierung, bestehen.

## Claims

1. A surgical suture having a filament (1) and possibly a needle (2) at at least one end of the filament, the suture having, in at least a first preshaped zone (3, 4) of its longitudinal expanse, at least one inherently stable convolution, sling or similar preshaped conformation, **characterized in that** the preshaped zone (3, 4) is generally in the form of a preshaped helix or spiral through which a zone of the filament or a filament end can be threaded or passed for loop and/or knot formation and that the suture has at least two preshaped zones (3, 4) in spaced relationship to each other, the preshaped helices or spirals of which have opposite directions of turn.

2. A surgical suture as claimed in claim 1, characterized in that the preshaped helix or spiral at least of one zone (3, 4), preferably of two zones (3, 4) of the suture in each case has a plurality of convolutions, suitably two.

3. A surgical suture as claimed in claim 1 or claim 2, characterized in that, for threading through a filament end, each helically or spirally preshaped zone (3, 4) of the suture is slightly larger than the diameter of the filament material and, possibly for passage of a surgical needle or filament holder, is adapted in its inside diameter (d) to the outside dimensions of said filament holder in the area of the forceps blades thereof.

4. A surgical suture according to the preamble of claim 1, characterized in that the suture has, at least in its first preshaped zone (3) of its longitudinal expanse, at least two converging convolutions, slings or similar preshaped conformations (19) having opposite directions of turn, that a further zone of the filament or a filament end (26, 27) can be passed through at least one of said convolutions or similar preshaped conformations (19) for loop and/or knot formation and that the central axes (20, 21) of said neighbouring, oppositely turned convolutions or similar preshaped conformations (19) are in spaced relationship to each another.

5. A surgical suture as claimed in claim 4, characterized in that in each case two neighbouring convolutions or similar preshaped conformations (19) with opposite directions of turn are approximately coplanar.

6. A surgical suture as claimed in claim 4, characterized in that in each case two neighbouring convolutions or similar preshaped conformations (19) with opposite directions of turn compose a preshaped, generally figure-eight form or double loop (22).

7. A surgical suture as claimed in claim 6, characterized in that the suture has two preshaped zones (3, 4) in spaced relationship to each other, the preshaped figure-eight forms or double loops (22) thereof having in particular opposite directions of turn.

8. A surgical suture as claimed in any one of claims 4 to 7, characterized in that of two neighbouring convolutions or similar preshaped conformations (19) having opposite directions of turn, the lead-in portion (23) of the one convolution (19a) and the lead-out portion (24) of the other convolution (19b) are arranged on opposite sides of their common transition area (25).

9. A surgical suture as claimed in any one of claims 4 to 8, characterized in that associated to every convolution or similar preshaped conformation (19) of the preshaped zone (3) having the one direction of turn, there is a further convolution (19) having an opposite direction of turn.

10. A surgical suture as claimed in any one of claims 4 to 9, characterized in that the preshaped zone (3) of the suture has more than two converging convolutions or similar preshaped conformations (19), that neighbouring, preshaped conformations (19) have opposite directions of turn and that the central axes (20, 21) of the preshaped conformations (19) having the same direction of turn are arranged approximately coaxially.

11. A surgical suture as claimed in any one of claims 4 to 10, characterized in that the preshaped zone (3) of the suture has three converging convolutions or similar preshaped conformations (19), wherein two preshaped conformations (19) having the same direction of turn enclose a preshaped conformation (19) having an opposite direction of turn therebetween.

12. A surgical suture as claimed in any one of claims 4 to 11, characterized in that at least one, preferably both of the outer convolutions or similar preshaped conformations (19) of the preshaped zone (3) in each case pass into a semi-circular cuvature (28) having an opposite direction of turn and that the suture portion (14, 15) adjoining the preshaped zone (3) is angled at that side of the curvature (28) which is averted from the transition area (29) provided between the convolution or similar preshaped conformation (19) and the curvature (28).

13. A surgical suture as claimed in any one of claims 4 to 12, characterized in that at least one, preferably both of the suture portions (14, 15) adjoining the preshaped zone (3) are angled crosswise, particularly at right angles to the plane formed by the neighbouring convolutions or similar preshaped conformations and/or double loops (22).

14. A surgical suture as claimed in any one of claims 4 to 13, characterized in that the suture portion(s) (14, 15) outside the convolutions or similar preshaped conformations are angled in a direction towards the adjoining preshaped zone (3, 4).

15. A surgical suture according to the preamble of claim 1, characterized in that the preshaped zone (3) has a generally figure-eight double loop (22) composed of a first convolution, sling or similar preshaped conformation (19a) and a second one (19b) with an opposite direction of turn, that the second conformation (19b) of said double loop (22) passes into a first curvature (28a) having the same direction of turn, that in that area of the double loop (22) which is averted from the first preshaped conformation (19a) the first curvature (28a) passes into a third convolution, sling or similar conformation (19c) having a direction of turn opposite that of the first curvature (28a), that the third preshaped conformation (19c) is adjoined by a second curvature (28b) having an opposite direction of turn, that the circular arc-shaped curvatures (28a, 28b) form the outline of a fourth sling whose opening at least locally overlaps that of the second conformation (19b) and that a zone of the filament or a filament end can be threaded or passed through said conformations (19) and sling openings for loop and/or knot formation.

16. A surgical suture as claimed in claim 15, characterized in that the second curvature (28b) is adjoined by a fifth convolution, sling or similar preshaped conformation (19e) having an opposite direction of turn.

17. A surgical suture as claimed in claim 15 or claim 16, characterized in that the central axes of the convolutions, slings or similar preshaped conformations (19) are approximately axially parallel and preferably coplanar and that the central axes of the second and fourth and possibly also of the first and fifth preshaped conformation are arranged approximately coaxially.

18. surgical suture as claimed in any one of claims 1 to 17, characterized in that the crossing portions of the transition areas formed by the convolutions, slings or similar preshaped conformations and/or curvatures are successively, evenly staggered in one direction along the longitudinal expanse of the suture.

19. A surgical suture as claimed in any one of claims 1 to 18, characterized in that the longitudinal or central axis (axes) (1, 20, 21) of the preshaped zone(s) (3, 4) is (are) arranged in the direction of the longitudinal expanse of the suture.

20. A surgical suture according to the preamble of claim 1, characterized in that the the suture has two neighbouring convolutions or similar preshaped conformations (19) or two neighbouring preshaped zones (3, 4) arranged with opposite directions of turn in a spectacle-like fashion and that a further zone of the filament or a filament end can be passed through said preshaped conformations or preshaped zones.

21. A surgical suture as claimed in claim 20, characterized in that the lead-in portion of the one convolution or similar preshaped conformation and the lead-out portion of the other convolution are arranged on the same side of their common transition area.

22. A surgical suture as claimed in any one of claims 1 to 21, characterized in that the preshaped zones of the suture, in particular the convolutions or similar preshaped conformations having the same direction of turn and/or the portions (5) provided between two neighbouring, preshaped zones and/or the suture portions leading to and/or away from the preshaped conformations have a signal colouring, signal coating or similar signal marking.

23. A surgical suture as claimed in any one of claims 1 to 22, characterized in that at least the preshaped zone (3, 4) thereof consists of elastic material.

24. A surgical suture as claimed in any one of claims 1 to 3, 7 to 14 and 20 to 23, characterized in that arranged between the spaced, preshaped zones (3, 4) is an undeformed suture portion (5).

25. A surgical suture as claimed in any one of claims 1 to 24, characterized in that the preshaped zone(s) (3, 4) is (are) adjoined preferably on both sides by an undeformed suture portion (14, 15) and that said portions (14, 15) point away from the preshaped zone(s) (3, 4).

26. A surgical suture as claimed in any one of claims 1 to 25, characterized in that the preshaped zone or zones of the suture take(s) the form of a segment or segments detachably connected to the adjacent filament or suture portion(s) (5, 14, 15) by means of a plug-type connector, clamp-type connector or similar fastener.

27. A surgical suture as claimed in any one of claims 1 to 26, characterized in that the filament (1) and/or the preshaped zone(s) (3, 4) of the suture consist of a memory metal, particularly a nickel-titanium metal alloy.

## Revendications

1. Matériel de suture chirurgicale avec un fil (1) et éventuellement une aiguille (2) à au moins une extrémité du fil, le matériel de suture présentant, dans au moins une première région partielle préformée (3, 4) de son développement longitudinal, au moins une spire, boucle ou préformage similaire de forme stable, **caractérisé** en ce que la région partielle préformée (3, 4) est un préformage approximativement en hélice ou en spirale, à travers lequel on peut enfiler ou faire passer une région partielle ou une extrémité du fil afin de former une boucle et/ou un noeud, et en ce que le matériel de suture possède aux moins deux régions partielles préformées (3, 4) mutuellement distantes, dont les préformages en hélice ou en spirale présentent des directions contraires de rotation.

2. Matériel de suture chirurgicale selon la revendication 1, **caractérisé** en ce que le préformage en hélice ou en spirale d'au moins une région partielle (3, 4), de préférence de deux régions partielles (3, 4) du matériel de suture, présente chaque fois plusieurs spires, opportunément deux spires.

3. Matériel de suture chirurgicale selon la revendication 1 ou 2, **caractérisé** en ce que chaque région partielle (3, 4) préformée en hélice ou en spirale du matériel de suture est, afin d'enfiler une extrémité du fil, légèrement plus grande que le diamètre du matériau du fil, et présente éventuellement, afin de faire passer un porte-aiguille ou porte-fil chirurgical, un diamètre intérieur (d) adapté aux dimensions extérieures de ce porte-fil dans la région des extrémités des mors de serrage de ce dernier.

4. Matériel de suture chirurgicale selon le préambule de la revendication 1, **caractérisé** en ce que le matériel de suture présente, au moins dans la première région partielle préformée (3) de son développement longitudinal, au moins deux spires, boucles ou préformages similaires (19) se raccordant les uns aux autres qui possèdent des directions contraires de rotation, en ce qu'on peut faire passer à travers au moins une de ces spires ou préformages similaires (19) une autre région partielle du fil ou une extrémité (26, 27) du fil afin de former une boucle ou un noeud, et en ce que les axes centraux (20, 21) de ces spires voisines à sens contraires ou préformages similaires (19) sont mutuellement distants.

5. Matériel de suture chirurgicale selon la revendication 4, **caractérisé** en ce que deux spires mutuellement voisines ou préformages similaires (19) de directions contraires de rotation sont chaque fois disposées approximativement dans un même plan.

6. Matériel de suture chirurgicale selon la revendication 4, **caractérisé** en ce que deux spires mutuellement voisines ou préformages similaires (19) de directions contraires de rotation forment chaque fois un préformage approximativement en huit ou encore une boucle double (22).

7. Matériel de suture chirurgicale selon la revendication 6, **caractérisé** en ce que le matériel de suture possède deux régions partielles préformées (3, 4) mutuellement distantes, dont les préformages en huit ou boucles doubles (22) présentent notamment des directions contraires de rotation.

8. Matériel de suture chirurgicale selon l'une des revendications 4 à 7, **caractérisé** en ce que, parmi deux spires mutuellement voisines ou préformages similaires (19) de directions contraires de rotation, la partie d'entrée (23) d'une spire (19a) et la partie de sortie (24) de l'autre spire (19b) sont disposées sur des côtés opposés de leur partie de raccordement commune (25).

9. Matériel de suture chirurgicale selon l'une des revendications 4 à 8, **caractérisé** en ce qu'à chaque spire ou préformage similaire (19), présentant une direction de rotation, de la région partielle préformée (3) est associée une autre spire (19) de direction contraire de rotation.

10. Matériel de suture chirurgicale selon l'une des revendications 4 à 9, **caractérisé** en ce que la région partielle préformée (3) du matériel de suture présente plus de deux spires ou préformages similaires (19) se raccordant les uns aux autres, en ce que des préformages (19) voisins présentent chaque fois des directions contraires de rotation, et en ce que les axes centraux (20, 21) des préformages (19) de même direction de rotation sont approximativement coaxiaux.

11. Matériel de suture chirurgicale selon l'une des revendications 4 à 10, **caractérisé** en ce que la région partielle préformée (3) du matériel de suture présente trois spires ou préformages similaires (19) se raccordant les uns aux autres, un préformage (19) de direction contraire de rotation étant inclus entre deux préformages (19) de même direction de rotation.

12. Matériel de suture chirurgicale selon l'une des revendications 4 à 11, **caractérisé** en ce qu'au moins une, et de préférence les deux spires extérieures ou préformages similaires (19) de la région partielle préformée (3), se raccordent chaque fois à une partie courbe semi-circulaire (28) de direction contraire de rotation, et en ce qu'à la suite de cette partie courbe, la partie (14, 15) du matériel de suture qui est respectivement attenante à la région partielle préformée (3), est coudée sur le côté de la partie courbe (28) qui est opposé à la partie de raccordement (29) prévue entre la spire ou préformage similaire (19) et la partie courbe (28).

13. Matériel de suture chirurgicale selon l'une des revendications 4 à 12, **caractérisé** en ce qu'au moins une, et de préférence les deux parties (14, 15) du matériel de suture attenantes à la région partielle préformée (3) sont coudées transversalement, et notamment perpendiculairement au plan formé par les spires voisines ou préformages similaires et/ou par les boucles doubles (22).

14. Matériel de suture chirurgicale selon l'une des revendications 4 à 13, **caractérisé** en ce que la ou les parties (14, 15) du matériel de suture, en dehors des spires ou préformages similaires, sont coudées en direction de la région partielle préformée attenante (3, 4).

15. Matériel de suture chirurgicale selon le préambule de la revendication 1, **caractérisé** en ce que la région partielle préformée (3) présente une boucle double (22) approximativement en forme de huit, qui est constituée d'une première spire, boucle ou préformage similaire (19a) ainsi que d'un deuxième préformage (19b) de direction contraire de rotation, en ce que le deuxième préformage (19b) de cette boucle double (22) se raccorde à une première partie courbe (28a) de même direction de rotation, en ce que la première partie courbe (28a) se raccorde, dans la région de la boucle double (22) qui est opposée au premier préformage (19a), à une troisième spire, bouche ou préformage similaire (19c) de direction de rotation contraire à celle de la première partie courbe (28a), en ce qu'au troisième préformage (19c) se raccorde une deuxième partie courbe (28b) de direction de rotation contraire à celle de ce dernier, en ce que les parties courbes en forme d'arc de cercle (28a, 28b) forment le contour d'une quatrième bouche dont l'ouverture chevauche au moins pour partie l'ouverture de bouche du deuxième préformage (19b), et en ce qu'une région partielle du fil ou une extrémité du fil peut être enfilée ou passée à travers ces préformages (19) et ouvertures de bouches afin de former une boucle et/ou un noeud.

16. Matériel de suture chirurgicale selon la revendication 15, **caractérisé** en ce qu'à la deuxième partie courbe (28b) se raccorde une cinquième spire, boucle ou préformage similaire (19e) de direction contraire de rotation.

17. Matériel de suture chirurgicale selon la revendication 15 ou 16, **caractérisé** en ce que les axes centraux des spires ou préformages similaires (19) s'étendent environ parallèlement entre eux et de préférence dans un même plan, et en ce que les axes centraux du deuxième et du quatrième préformage, ainsi que du premier et du cinquième préformage le cas échéant, sont approximativement coaxiaux.

18. Matériel de suture chirurgicale selon l'une des revendications 1 à 17, **caractérisé** en ce que les spires ou préformages similaires, et/ou les parties de raccordement formées par des parties courbes, sont disposés avec des régions de croisement qui, dans le développement longitudinal du matériel de suture, se succèdent en étant mutuellement décalées dans une direction de façon régulière.

19. Matériel de suture chirurgicale selon l'une des revendications 1 à 18, **caractérisé** en ce que le ou les axes centraux ou longitudinaux (1, 20, 21) de la ou des régions partielles préformées (3, 4) sont disposés dans la direction du développement longitudinal du matériel de suture.

20. Matériel de suture chirurgicale selon le préambule de la revendication 1, **caractérisé** en ce que le matériel de suture présente deux spires mutuellement voisines ou préformages similaires (19) ou deux régions partielles préformées (3, 4) voisines qui sont disposées l'une part rapport à l'autre avec des directions contraires de rotation en formant des lunettes, et en ce qu'une autre région partielle du fil ou une extrémité du fil peut être enfilée à travers ces préformages ou régions partielles préformées.

21. Matériel de suture chirurgicale selon la revendication 20, **caractérisé** en ce que la partie d'entrée d'une spire ou préformage similaire et la partie de sortie de l'autre spire sont disposées sur le même côté de leur partie commune de raccordement.

22. Matériel de suture chirurgicale selon l'une des revendications 1 à 21, **caractérisé** en ce que les régions partielles préformées du matériel de suture, notamment les spires ou préformages similaires de même direction de rotation, et/ou les parties (5) prévues entre deux régions partielles préformées voisines, et/ou les parties du matériel de suture précédant ou suivant les préformages, présentent une coloration, enduction ou marquage similaire de signalisation.

23. Matériel de suture chirurgicale selon l'une des revendications 1 à 22, **caractérisé** en ce qu'au moins sa région partielle préformée (3, 4) est réalisée en matériau élastique.

24. Matériel de suture chirurgicale selon l'une des revendications 1 à 3, 7 à 14 et 20 à 23, **caractérisé** en ce qu'une partie non déformée (5) du matériel de suture est disposée entre les régions partielles préformées (3, 4) mutuellement distantes.

25. Matériel de suture chirurgicale selon l'une des revendications 1 à 24, **caractérisé** en ce qu'une partie non déformée (14, 15) du matériel de suture se raccorde de préférence de part et d'autre à la ou les régions partielles préformées (3, 4), et en ce que ces parties (14, 15) sont orientées en éloignement de la ou les régions partielles préformées (3, 4).

26. Matériel de suture chirurgicale selon l'une des revendications 1 à 25, **caractérisé** en ce que la ou les régions partielles préformées du matériel de suture sont réalisées sous la forme d'éléments séparés, qui sont assemblés de manière détachable à la ou les parties attenantes (5, 14, 15) du fil ou matériel de suture au moyen d'un élément d'assemblage par emboîtement, par serrage ou similaire.

27. Matériel de suture chirurgicale selon l'une des revendications 1 à 26, **caractérisé** en ce que le fil (1) et/ou la ou les régions partielles préformées (3, 4) du matériel de suture sont réalisés en un matériau métallique à mémoire, notamment un alliage métallique de nickel et de titane.
